# EUROPÄISCHE PATENTSCHRIFT

(11) **EP 1 416 983 B1**
(45) Veröffentlichungstag und Bekanntmachung des Hinweises auf die Patenterteilung: **01.08.2007**
(21) Anmeldenummer: 02745038.6
(22) Anmeldetag: 22.07.2002
(51) Int. Cl.: A61M 5/00, A61M 5/315

(54) **Verbindung von Gehäuseabschnitten eines Verabreichungsgeräts für eine dosierte Verabreichung eines ausschüttbaren Produkts**
Connection of housing sections of an administration device for dosed administration of a distributable product
Assemblage de sections de boîtier d'un appareil d'administration de produit pour administrer de manière dosée un produit déversable

(30) Priorität: 30.07.2001 DE 20112501 U; 21.12.2001 DE 10163329
(43) Veröffentlichungstag der Anmeldung: 12.05.2004
(73) Patentinhaber: TecPharma Licensing AG, 3401 Burgdorf (CH)
(72) Erfinder: KIRCHHOFER, Fritz, CH-3454 Sumiswald (CH); GRAF, Roney, CH-3400 Burgdorf (CH)
(74) Vertreter: Schwabe - Sandmair - Marx
(86) Internationale Anmeldenummer: PCT/CH2002/000412
(87) Internationale Veröffentlichungsnummer: WO 2003/011373

(56) Entgegenhaltungen:
- EP-A- 0 498 737
- WO-A-95/32749
- WO-A-97/17095
- DE-A- 4 425 763
- US-A- 4 973 318
- US-A- 5 611 783

## Beschreibung

Die Erfindung betrifft bei Produktverabreichungsgeräten mit wenigstens zwei Gehäuseabschnitten die Verbindung dieser beiden Gehäuseabschnitte. Bevorzugte Beispiele erfindungsgemäßer Geräte sind Injektionsgeräte, insbesondere Injektionspens, besonders bevorzugte Beispiele sind semidisposable Pens. Das Gerät kann auch einen Dosierteil eines Inhalationsgeräts oder eines Geräts für eine orale Einnahme oder eine anders geartete Art der Verabreichung eines fluiden Produkts bilden.

Die WO 97/17095 beschreibt ein Injektionsgerät, das aus einem Dosier- und Betätigungsmodul und einem Reservoirmodul besteht, die lösbar miteinander verbunden sind. Das Reservoirmodul ist als Einwegmodul konzipiert, während das Dosier- und Betätigungsmodul nach einem Verbrauch des Reservoirmoduls für eine Wiederverwendung mit einem neuen Reservoirmodul bestimmt ist. Das Reservoirmodul enthält ein Reservoir für ein zu injizierendes Produkt und lagert eine Kolbenstange, die für eine Produktausschüttung auf einen in dem Reservoir aufgenommenen Kolben wirkt. Die Kolbenstange weist ein Außengewinde auf, das mit einem Innengewinde eines Dosiseinstellglieds in einem Gewindeeingriff steht. Die Kolbenstange ist linear geführt, so dass bei einer Drehbewegung des Dosiseinstellglieds die Kolbenstange in der eine Vorschubrichtung auf den Kolben zu bewegt und dadurch ein lichter Abstand zwischen einem vorderen Ende der Kolbenstange und den Kolben verändert wird. Das Dosier- und Betätigungsmodul und das Reservoirmodul werden durch Aufschrauben oder Aufklicken miteinander verbunden.

Ein Vorteil des Konzepts des semidisposable Injektionsgeräts ist, dass an der Dosierung und Ausschüttung beteiligte Teile des Geräts nur für die Ausschüttung eines einzigen Reservoirinhalts ausgelegt werden müssen. Da bei wiederholter Verwendung solche Teile stets wieder in einen Anfangszustand zurückgeführt werden müssten, wären sie ferner einem nicht zu unterschätzenden Beschädigungsrisiko ausgesetzt. Die Sicherheit der korrekten Dosisauswahl und Dosisausschüttung muss daher bei semidisposable Injektionsgeräten nicht geringer sein als bei vollständig wiederverwendbaren Geräten. Darüber hinaus ist der Austausch eines kompletten Reservoirmoduls einfacher als der Austausch nur eines Reservoirs.

Aus der DE 44 25 763 A1 ist eine Vorrichtung zum Vorschub eines Kolbenverschiebeorgans bekannt, das zum Vorschub eines Kolbens in einem Zylinder bestimmt ist. Eine Sicherung gegen ein ungewolltes Verschieben des Vorschuborgans ist vorgesehen, die mit einer Feder als Sperrorgan ausgerüstet ist. Ein zu verabreichendes Medikament ist in einer Kartusche vorgesehen und ein Kolben in der Kartusche wird über eine Zahnstange vorangetrieben, die über einen Druckknopf betätigt werden kann. Die Zahnstange wird über eine Vorschubhülse vorangetrieben, wobei durch die Zahnteilung der Zahnstange die einzelnen Vorschubschritte für den Kolben unabänderlich vorgegeben sind.

Aus der WO95/32749 ist ein Verabreichungsgerät bekannt, das die Merkmale im Oberbegriff des Anspruchs 1 umfasst. Jedoch ist hier ein Dosiseinstellglied von außen zugänglich und die Dosis wird dadurch eingestellt, dass die beiden außen liegenden Gehäuseteile zueinander mehr oder weniger beabstandet werden, je nachdem ob eine größere oder kleinere Dosis zu verabreichen ist. Die Dosierung und die Verabreichung wird hier über unterschiedliche Mechaniken vollbracht, die lediglich indirekt miteinander verkoppelt sind.

Probleme kann der Zusammenbau des Dosier- und Betätigungsmoduls mit einem neuen Reservoirmodul dennoch aufwerfen, insbesondere im Hinblick auf die Korrektheit der Dosisauswahl für die nach dem Zusammenbau vorzunehmende erste Produktausschüttung. Wegen der zum Zwecke der Dosierung und Ausschüttung erforderlichen Kopplung von Kolbenstange, Dosiseinstellglied und dem Dosier- und Betätigungsmodul besteht die Gefahr, dass durch den Zusammenbau die Position des Dosiseinstellglieds oder gar der Kolbenstange durch den Zusammenbau von Reservoirmodul und Dosier- und Betätigungsmodul in nicht vorhersehbarer Weise beeinflusst wird. Das Problem kann bei jeder Dosier- und Ausschüttmechanik auftreten, bei der die Vorgänge der Dosierung und der Ausschüttung separat durchgeführt werden, wie dies bei Injektionsgeräten der Fall ist. Das Problem ist auch nicht auf semidisposable Pens beschränkt. Mit dem Problem ist vielmehr jedes Verabreichungsgerät behaftet, bei dem durch eine einfache Verbindung von zwei Gehäuseabschnitten die für die Dosierung und Ausschüttung erforderliche Kopplung von Kolbenstange oder eines anderen Abtriebsglieds, Dosiseinstellglied und einer Dosier- und Antriebsvorrichtung hergestellt wird. Darüber hinaus besteht der Wunsch, insbesondere in der Selbstverabreichung des Produkts, nach Einfachheit in der Handhabung, wobei eine einfache Handhabung nicht nur in vorteilhafter Weise den Handhabungskomfort, sondern auch die Sicherheit erhöht.

Bei einem Verabreichungsgerät mit wenigstens zwei Gehäuseabschnitten und einer Dosier- und Ausschüttmechanik, deren Teile durch den Zusammenbau der Gehäuseabschnitte miteinander gekoppelt werden, ist es eine Aufgabe der Erfindung, durch den Zusammenbau dieser Gehäuseabschnitte einen definierten Ausgangszustand der Dosier- und Ausschüttmechanik zu erhalten und den Zusammenbau dennoch zu vereinfachen.

Die Erfindung betrifft die Verbindung eines vorderen Gehäuseabschnitts und eines hinteren Gehäuseabschnitts bei einem Verabreichungsgerät, vorzugsweise Injektionsgerät, das die Verabreichung eines Produkts in einer auswählbaren Dosis oder mehreren auswählbaren Dosen erlaubt. Das Injektionsgerät umfasst demgemäß den vorderen Gehäuseabschnitt und den hinteren Gehäuseabschnitt. Das Produkt ist in einem Reservoir enthalten. Der vordere Gehäuseabschnitt kann das Reservoir unmittelbar bilden, bevorzugter bildet jedoch ein von dem vorderen Gehäuseabschnitt aufgenommenes Behältnis, beispielsweise eine handelsübliche Ampulle, das Reservoir.

Das Verabreichungsgerät umfasst ferner ein Abtriebsglied, das von wenigstens einem der Gehäuseabschnitte, vorzugsweise dem vorderen Gehäuseabschnitt, so gelagert wird, dass es eine Ausschüttbewegung ausführen kann, durch die eine zuvor ausgewählte Produktdosis aus dem Reservoir ausgeschüttet wird. Da die Produktausschüttung üblicherweise durch die Bewegung eines in dem Reservoir aufgenommenen Kolbens in eine Vorschubrichtung auf einen Auslass des Reservoirs zu vorgenommen wird, bildet vorzugsweise eine Kolbenstange das Abtriebsglied einer Dosier- und Ausschüttmechanik des Verabreichüngsgeräts. Das Abtriebsglied kann mit dem Kolben fest, d.h. ständig, verbunden sein, worunter auch eine einstückige Ausbildung von Kolben und Abtriebsglied verstanden werden soll. In bevorzugter Ausführung sind der Kolben und das Abtriebsglied jedoch als separate Bauteile ausgeführt, und es drückt zum Zwecke der Produktausschüttung das Abtriebsglied mit einem vorderen Ende gegen eine Rückseite des Kolbens.

Des Weiteren gehört zum Verabreichungsgerät ein Dosiseinstellglied, das der Auswahl der Produktdosis dient und zu diesem Zweck relativ zu dem Abtriebsglied und vorzugsweise auch zu dem vorderen Gehäuseabschnitt eine Dosierbewegung ausführt. Wird die Produktausschüttung mittels Kolben und Kolbenstange bewirkt, so wird durch die Dosierbewegung des Dosiseinstellglieds der maximale Hub der Kolbenstange für deren Ausschüttbewegung eingestellt.

Schließlich umfasst das Injektionsgerät eine Dosier- und Antriebsvorrichtung, die relativ zu dem vorderen Gehäuseabschnitt translatorisch und um eine Rotationsachse rotatorisch bewegbar ist. Die Dosier- und Antriebsvorrichtung ist vor dem Zusammenbau der Gehäuseabschnitte mit dem hinteren Gehäuseabschnitt verbunden. Der hintere Gehäuseabschnitt kann ein Dosierelement der Dosier- und Antriebsvorrichtung bilden, indem er in einer Verbindungsendstellung, die er nach dem Zusammenbau der Gehäuseabschnitte einnimmt, d.h. nach der Herstellung der Verbindung, relativ zu dem vorderen Gehäuseabschnitt um eine gemeinsame Längsachse drehbar ist. Bevorzugterweise ist der hintere Gehäuseabschnitt jedoch in der Verbindungsendstellung relativ zu dem vorderen Gehäuseabschnitt rotatorisch und vorzugsweise auch translatorisch nicht bewegbar fixiert und übernimmt dementsprechend keine Dosierfunktion. Die Dosier- und Antriebsvorrichtung wird bei der Herstellung der Verbindung zwischen dem vorderen und dem hinteren Gehäuseabschnitt, vorzugsweise unmittelbar durch die Herstellung der Verbindung selbst, mit dem Abtriebsglied und dem Dosiseinstellglied so gekoppelt, dass nach der Herstellung der Verbindung eine Rotationsbewegung der Dosier- und Antriebsvorrichtung die Dosierbewegung des Dosiseinstellglieds und eine Translationsbewegung der Dosier- und Antriebsvorrichtung die Ausschüttbewegung des Abtriebsglieds bewirkt.

Die Kopplung von Abtriebsglied, Dosiseinstellglied und Dosier- und Antriebsvorrichtung kann durch einen unmittelbaren paarweisen Eingriff von je zwei dieser Komponenten ohne Zwischenschaltung von Übertragungsgliedern erfolgen, wie dies bevorzugt wird, ohne dass hierdurch die Zwischenschaltung von einem oder mehreren anderen Übertragungsgliedern ausgeschlossen werden soll. Das Dosiseinstellglied ist mit dem Abtriebsglied und wenigstens einem der Gehäuseabschnitte, vorzugsweise dem vorderen Gehäuseabschnitt, bevorzugt so gekoppelt, dass es nur gemeinsam mit dem Abtriebsglied die Ausschüttbewegung ausführt und durch die Rotationsbewegung der Dosier- und Antriebsvorrichtung relativ zu dem Abtriebsglied entgegen der Vorschubrichtung bewegt wird. Ein unmittelbarer Eingriff mit dem Abtriebsglied ist vorzugsweise ein Gewindeeingriff.

Die Rotationsbewegung der Dosier- und Antriebsvorrichtung wird vorzugsweise in eine Rotationsbewegung des Dosiseinstellglieds oder des Abtriebsglieds um die gleiche Rotationsachse übertragen, um die Dosierbewegung des Dosiseinstellglieds zu erhalten. Die Dosierbewegung des Dosiseinstellglieds kann in bevorzugten Ausführungen eine kombinierte Translations- und Rotationsbewegung oder eine reine Translationsbewegung sein. Die Ausschüttbewegung des Abtriebsglieds ist vorzugsweise eine Translationsbewegung in die gleiche Richtung und besonders bevorzugt entlang der gleichen Translationsachse wie die Translationsbewegung der Dosier- und Antriebsvorrichtung. Im Folgenden wird die Rotationsbewegung der Dosier- und Antriebsvorrichtung ebenfalls als Dosierbewegung und ihre Translationsbewegung auch als Ausschüttbewegung bezeichnet. Besonders bevorzugt sind die Rotationsachse und die Translationsachse der Dosier- und Antriebsvorrichtung identisch. Eine translatorische Bewegung des Dosierglieds erfolgt vorzugsweise ebenfalls entlang dieser Achse.

In einer bevorzugten ersten Ausführungsform ist die Dosier- und Antriebsvorrichtung in einem unmittelbaren, verdrehgesicherten Eingriff mit dem Abtriebsglied, um die Rotationsbewegung zum Zwecke der Dosisauswahl zunächst auf das Abtriebsglied und über das Abtriebsglied auf das Dosiseinstellglied zu übertragen, so dass das Dosiseinstellglied seine Dosierbewegung ausführt. In einer besonders bevorzugten zweiten Ausführungsform sind zum Zwecke der Dosierung die Dosier- und Antriebsvorrichtung und das Dosiseinstellglied unmittelbar in einem verdrehgesicherten Eingriff, d.h. es macht in diesem Fall das Dosiseinstellglied die Rotationsbewegung der Dosier- und Antriebsvorrichtung mit, so dass seine Dosierbewegung sich aus einer Rotations- und einer Translationsbewegung zusammensetzt. In beiden Ausführungsformen sind das Abtriebsglied und das Dosiseinstellglied derart gekoppelt, vorzugsweise unmittelbar miteinander in Eingriff, dass sie die Ausschüttbewegung gemeinsam ausführen.

Nach der Erfindung sind an einem der Gehäuseabschnitte wenigstens eine Axialführung und an dem anderen der Gehäuseabschnitte wenigstens ein Eingriffselement gebildet. Die wenigstens eine Axialführung und das wenigstens eine Eingriffselement greifen bei der Herstellung der Verbindung der Gehäuseabschnitte und vorzugsweise auch noch nach der Herstellung der Verbindung ineinander, um eine Linearführung für die Gehäuseabschnitte zu bilden. Die Linearführung stellt sicher, dass die Gehäuseabschnitte bei der Herstellung ihrer Verbindung bis in eine Verbindungsendstellung relativ zueinander in Bezug auf die Rotationsachse der Dosier- und Antriebsvorrichtung und vorzugsweise absolut verdrehgesichert aufeinandergeschoben werden. Vorzugsweise ist außer der Bewegung des Aufeinanderschiebens zwischen den Gehäuseabschnitten keinerlei Relativbewegung möglich. Das axial linear geführte Aufeinanderschieben der Gehäuseabschnitte macht den Zusammenbau des Geräts besonders einfach. Ferner wird verhindert, dass bei der Herstellung der Kopplung der Dosier- und Antriebsvorrichtung mit dem Abtriebsglied und dem Dosiseinstellglied durch unbeabsichtigte Rotationsbewegungen der Gehäuseabschnitte relativ zueinander unerwünschte Rotationsbewegungen auf das Abtriebsglied und/oder das Dosiseinstellglied übertragen werden, die eine Dosierbewegung des Dosiseinstellglieds verursachen könnten, und sei es auch nur aufgrund von Reaktionsbewegungen der Dosier- und Antriebsvorrichtung auf solche unbeabsichtigten Rotationsbewegungen zwischen den Gehäuseabschnitten.

Falls der vordere Gehäuseabschnitt und der hintere Gehäuseabschnitt zusammen eine Verriegelungseinrichtung bilden, umfasst diese Verriegelungseinrichtung vorzugsweise eine Verriegelungssperre, die eine Verriegelung der beiden Gehäuseabschnitte aneinander nur in einer vorderen Endposition der Dosier- und Antriebsvorrichtung zulässt. In diesem Fall wird durch die erfindungsgemäße Axialführung der Gehäuseabschnitte aneinander als weiterer Vorteil erhalten, dass die Verriegelung nicht dadurch verhindert werden kann, dass bei dem Zusammenbau das Dosiseinstellglied durch die Herstellung der Kopplung bewegt wird. Letzteres könnte nämlich einen Einfluss auf die vordere Endposition der Dosier- und Antriebsvorrichtung haben.

Für die Linearführung der Gehäuseabschnitte ist die wenigstens eine Axialführung vorzugsweise unmittelbar an, d.h. in oder auf einer Mantelfläche des einen der Gehäuseabschnitte gebildet. Besonders bevorzugt bildet die Axialführung einen Führungskanal für das wenigstens eine Eingriffsglied des anderen der Gehäuseabschnitte. Vorzugsweise ist das wenigstens eine Eingriffsglied in dem Führungskanal beidseits eng gleitgeführt. Auch das wenigstens eine Eingriffsglied ist vorzugsweise unmittelbar an, d.h. in oder auf einer Mantelfläche des anderen der Gehäuseabschnitte als axial kurzer Eingriffsnocken oder als axial gestreckte Eingriffsrippe geformt.

Vorteilhaft ist es, wenn in Umfangsrichtung voneinander beabstandet mehrere Axialführungen an der Mantelfläche des einen der Gehäuseabschnitte gebildet sind. Die mehreren Axialführungen sind zweckmäßigerweise gleichmäßig über den Umfang der Mantelfläche verteilt angeordnet. Die Ausbildung mehrerer Axialführungen, insbesondere die Ausbildung von mehreren gleichmäßig über den Umfang verteilt angeordnete Axialführungen, erleichtert die Ausrichtung der Gehäuseabschnitte, da die Gehäuseabschnitte in einer Mehrzahl von vorgegebenen Drehwinkelpositionen aufeinandergeschoben werden können. Lediglich der Vollständigkeit wegen sei darauf hingewiesen, dass auch mehrere Eingriffselemente an dem anderen der Gehäuseabschnitte vorgesehen sein können, insbesondere mehrere Eingriffselemente in ebenfalls über den Umfang gleichmäßiger Verteilung.

Um die Ausrichtung der beiden Gehäuseabschnitte in Bezug auf ihre Drehwinkelposition relativ zueinander zu erleichtern, kann ein von der wenigstens einen Axialführung gebildeter Führungskanal an seinem Ende, das bei der Ausrichtung dem anderen der Gehäuseabschnitte zugewandt ist, in einer trichterförmigen Verbreiterung auslaufen. Falls zwei nebeneinander angeordnete vorstehende Abschnitte, beispielsweise Rippen, mit ihren einander zugewandten Seitenwänden je eine Axialführung bilden, laufen diese vorstehenden Abschnitte axial verjüngt aus, um so die trichterförmige Verbreiterung zu bilden. Besonders vorteilhaft ist es ferner, wenn solche vorstehenden Abschnitte an ihrem Ende, das dem anderen der Gehäuseabschnitte zugewandt ist, in radialer Richtung zur Mantelfläche ihres Gehäuseabschnitts hin verjüngt auslaufen. Eine kombinierte axiale und radiale Verjüngung am Einlauf der Axialführung, insbesondere in einen Führungskanal, erleichtert die Ausrichtung der Gehäuseabschnitte besonders. Falls an dem einen der Gehäuseabschnitte mehrere Axialführungen vorgesehen sind, sind vorzugsweise mehrere und besonders bevorzugt alle Axialführungen axial und vorzugsweise auch radial verjüngt ausgebildet.

Das vorstehend in Bezug auf die eine oder die mehreren Axialführungen, Gesagte gilt ebenso auch für das wenigstens eine oder die mehreren Eingriffselemente des anderen Gehäuseabschnitts.

Falls vor dem Verbinden der Gehäuseabschnitte das Dosiseinstellglied mit dem vorderen Gehäuseabschnitt und die Dosier- und Antriebsvorrichtung mit dem hinteren Gehäuseabschnitt verbunden sind, wie dies bevorzugt wird, so werden das Dosiseinstellglied und ein Dosierelement oder Dosier- und Antriebselement der Dosier- und Antriebsvorrichtung von ihrem jeweiligen Gehäuseabschnitt vorzugsweise in vorgegebenen Drehwinkelpositionen relativ zu dem jeweiligen Gehäuseabschnitt gehalten. Besonders bevorzugt werden sie in der jeweiligen Drehwinkelposition von ihrem Gehäuseabschnitt axial lineargeführt.

Das Dosierelement oder Dosier- und Antriebselement wird von dem hinteren Gehäuseabschnitt in seinen diskreten Drehwinkelpositionen durch einen lösbaren Eingriff, vorzugsweise einen Rasteingriff, gehalten. Durch den Eingriff wird bevorzugterweise auch gleichzeitig ein Klickgeräusch erzeugt, wenn das Dosierelement oder Dosier- und Antriebselement bei seiner Dosierbewegung aus der einen Drehwinkelposition in die nächste bewegt wird, so dass der Dosiervorgang auch akustisch angezeigt wird.

Das Dosiseinstellglied kann von dem vorderen Gehäuseabschnitt in einem unlösbaren Führungseingriff lineargeführt sein. Falls die Dosierbewegung des Dosiseinstellglieds eine kombinierte Rotations- und Translationsbewegung ist, muss die Fixierung in vorgegebenen Drehwinkelpositionen natürlich lösbar sein. In diesem Fall ist der Eingriff zwischen dem Dosiseinstellglied und dem vorderen Gehäuseabschnitt vorzugsweise ebenfalls ein Rasteingriff. Die vorgegebenen Drehwinkelpositionen des Dosiseinstellglieds und des Dosierelements oder Dosier- und Antriebselements sind auf die Axialführung der Gehäuseabschnitte abgestimmt, so dass in jeder der derart vorgegebenen Drehwinkelpositionen des Dosiseinstellglieds und des Dosierelements oder Dosier- und Antriebselements bei dem axialen Aufeinanderschieben der Gehäuseabschnitte die Kopplung von Abtriebsglied, Dosiseinstellglied und Dosier- und Antriebsvorrichtung verdrehfrei hergestellt wird.

Die Dosier- und Antriebsvorrichtung kann manuell, halb automatisch oder voll automatisch arbeiten. Im ersten Fall wird sowohl die rotatorische Dosierbewegung als auch die translatorische Ausschüttbewegung manuell ausgeführt. Im zweiten Fall wird entweder die Dosierbewegung oder die Ausschüttbewegung manuell ausgeführt, und die andere Bewegung wird motorisch oder mittels einer anderen Art der Kraftbeaufschlagung, beispielsweise mittels Federkraft bewirkt, wenn der Verwender die entsprechende Bewegung durch einen Betätigungshandgriff ausgelöst hat. Im dritten Fall, der vollautomatischen Dosier- und Antriebsvorrichtung werden die Dosierbewegung und die Ausschüttbewegung motorisch oder mittels einer anderen Kraft, beispielsweise Federkraft, bewirkt. Es wird in diesem Fall manuell lediglich die Dosis ausgewählt, beispielsweise mittels einer oder mehreren Tasten, und es wird die Ausschüttbewegung ebenfalls durch einen eigenen, entsprechenden Betätigungshandgriff des Verwenders ausgelöst. In den meisten Ausführungen ist das erfindungsgemäße Verabreichungsgerät mit einer manuellen Dosier- und Antriebsvorrichtung ausgerüstet, die dann als Dosier- und Betätigungsvorrichtung bezeichnet wird. Soweit daher von einer Dosier- und Betätigungsvorrichtung die Rede ist, wird damit die manuelle Ausführung bezeichnet. Soweit von einer Dosier- und Antriebsvorrichtung die Rede ist, soll hierdurch keine Beschränkung in Bezug auf manuell, halb automatisch oder voll automatisch vorgenommen werden, sondern es soll jede der Ausführungen umfasst sein. Der Begriff "Dosier- und Betätigungsmodul" wird jedoch im Zusammenhang mit sämtlichen Ausführungen der Dosier- und Antriebsvorrichtung verwendet.

Die Dosier- und Antriebsvorrichtung kann ein Dosierelement, das die Dosierbewegung ausführt, und separat ein Antriebselement aufweisen, das die Ausschüttbewegung ausführt. Vorzugsweise werden die Dosierbewegung und Ausschüttbewegung jedoch von dem gleichen Körper der Dosier- und Antriebsvorrichtung ausgeführt, der im Folgenden daher auch als Dosier- und Antriebselement oder als Dosier- und Betätigungselement bezeichnet wird.

Bei dem Produkt handelt es sich vorzugsweise um ein Fluid, besonders bevorzugt eine Flüssigkeit, für eine medizinische, therapeutische, diagnostische, pharmazeutische oder kosmetische Anwendung. Das Produkt kann beispielsweise Insulin, ein Wachstumshormon oder auch dünn- bis dickflüssige, breiförmige Nahrung sein. Das Verabreichungsgerät wird vorzugsweise in solchen Verwendungen eingesetzt, in denen ein Verwender sich das Produkt selbst verabreicht, wie es beispielsweise in der Diabetestherapie üblich ist. Die Verwendung im stationären und ambulanten Bereich durch geschultes Personal soll jedoch nicht ausgeschlossen sein.

Die Produktverabreichung kann bei einem Injektionsgerät mittels Injektionskanüle oder beispielsweise einer Düse für nadellose Injektionen erfolgen. Die Injektion oder Infusion kann insbesondere subkutan oder venös vorgenommen werden, oder auch intramuskulär. Im Falle der Verabreichung per Inhalation kann die ausgewählte Produktdosis aus dem Reservoir beispielsweise in eine Kammer des Inhalationsgeräts ausgeschüttet und mittels einer Zerstäubungseinrichtung für die Inhalation zerstäubt werden. Denkbar ist ferner eine orale Einnahme oder eine Verabreichung über die Speiseröhre, um nur einige Beispiele der Verabreichung zu nennen.

Besonders bevorzugt ist das Verabreichungsgerät semidisposable. In diesem Fall ist der vordere Gehäuseabschnitt Träger eines Reservoirmoduls, das nach Entleerung des Reservoirs entsorgt oder in einen Kreislauf zurückgeführt wird, und der hintere Gehäuseabschnitt ist Träger eines Dosier- und Betätigungsmoduls, das in Verbindung mit einem neuen Reservoirmodul wiederholt verwendbar ist. Da das Reservoirmodul als Verbrauchsmodul auch separat gehandelt wird, ist es auch separat Gegenstand der Erfindung. Auch das Dosier- und Betätigungsmodul kann separat Gegenstand der Erfindung sein. Ebenso bildet ein System aus einem Verabreichungsgerät und wenigstens einem Reservoirmodul, das das Reservoirmodul des Geräts nach Verbrauch ersetzen kann, einen Gegenstand der Erfindung. Das Konzept des zweigeteilten Verabreichungsgeräts in einen für die nur einmalige Verwendung vorgesehenen Teil und einen für die wiederholte Verwendung vorgesehenen Teil (semidisposable) ist vorteilhaft insbesondere für Injektionspens, aber desweiteren auch für beispielsweise Inhalationsgeräte oder Geräte für die orale Einnahme eines Produkts oder eine künstliche Ernährung.

In den Unteransprüchen werden weitere bevorzugte Ausgestaltungen der Erfindung beschrieben, wobei Merkmale die nur in Bezug auf das Verabreichungsgerät oder nur in Bezug auf ein Reservoirmodul oder ein Dosier- und Betätigungsmodul beansprucht sind, auch bevorzugte Merkmale in Bezug auf den jeweils anderen Anspruchsgegenstand sind.

Ausführungsbeispiele der Erfindung werden nachfolgend anhand von Figuren beschrieben. An den Ausführungsbeispielen offenbar werdende Merkmale bilden je einzeln und in jeder Merkmalskombination die Gegenstände der Ansprüche vorteilhaft weiter. Auch Merkmale, die nur an dem einen Beispiel offenbart sind, bilden das jeweils andere Beispiel weiter oder zeigen eine Alternative auf, soweit nichts Gegenteiliges offenbart wird oder nur der Fall sein kann. Es zeigen:
- Figur 1: zwei Teile eines Reservoirmoduls nach einem ersten Ausführungsbeispiel,
- Figur 2: das aus den zwei Teilen der Figur 1 erhaltene Reservoirmodul,
- Figur 3: ein das Reservoirmodul der Figur 2 umfassendes Injektionsgerät nach dem ersten Ausführungsbeispiel in einem Längsschnitt,
- Figur 4: einen Teil des Injektionsgeräts der Figur 3,
- Figur 5: einen Mechanikhalter des Reservoirmoduls in einem Längsschnitt und zwei Ansichten,
- Figur 6: eine von dem Mechanikhalter gelagerte Blockiereinrichtung für eine Kolbenstange,
- Figur 7: eine Kolbenstange in einem Längsschnitt und einer Stirnansicht,
- Figur 8: eine Verriegelungssperre in einem Längsschnitt, einer Ansicht und einer Draufsicht,
- Figur 9: ein zweites Ausführungsbeispiel eines Injektionsgeräts,
- Figur 10: den Querschnitt A-A der Figur 9,
- Figur 11: den Querschnitt B-B der Figur 9,
- Figur 12: den Querschnitt C-C der Figur 9,
- Figur 13: den Querschnitt D-D der Figur 9,
- Figur 14: den Mechanikhalter des zweiten Ausführungsbeispiels in einer perspektivischen Darstellung,
- Figur 15: den Mechanikhalter der Figur 14 in einer Ansicht,
- Figur 16: den Querschnitt A-A der Figur 15,
- Figur 17: das Dosiseinstellglied des zweiten Ausführungsbeispiels in einer perspektivischen Darstellung,
- Figur 18: das Dosiseinstellglied der Figur 17 in einem Längsschnitt,
- Figur 19: das Dosiseinstellglied der Figur 17 in einer Ansicht,
- Figur 20: das Dosiseinstellglied der Figur 17 in einer Draufsicht,
- Figur 21: einen Teil des Injektionsgeräts nach Figur 3 und
- Figur 22: einen Teil des Injektionsgeräts nach Figur 9.

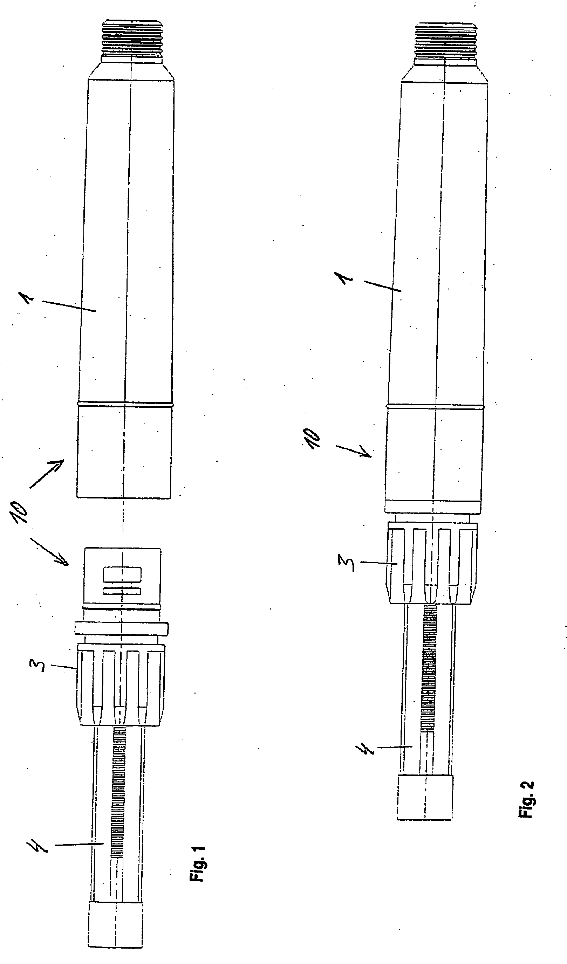

Figur 1 zeigt in einer Ansicht ein Reservoirteil 1 und einen Mechanikhalter 3, die miteinander verbunden werden, um das in Figur 2 dargestellte Reservoirmodul 10 zu bilden.

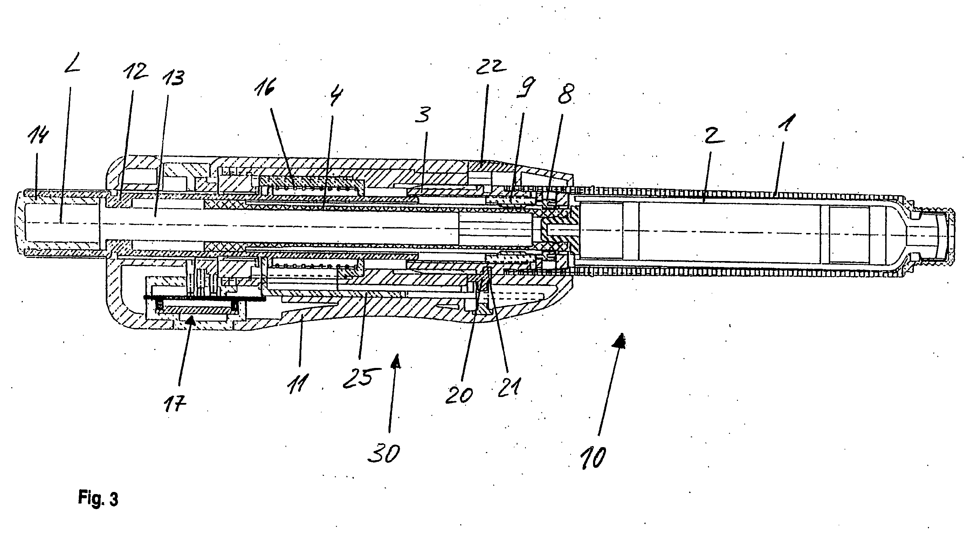

In den Figuren 1 und 2 ist ferner eine Kolbenstange 4 erkennbar, die an einem von dem Reservoirteil 1 abgewandten Ende des Mechanikhalters 3 in den Mechanikhalter 3 hineinragt und von dem Mechanikhalter 3 in eine in Längsachse L der Kolbenstange 4 weisende Vorschubrichtung auf ein von dem Mechanikhalter 3 abgewandtes, vorderes Ende des Reservoirteils 1 zu verschiebbar gelagert wird. Das Reservoirteil 1 ist im Wesentlichen ein im Querschnitt kreisförmiger Hohlzylinder, der an seinem vorderen Ende einen Verbindungsbereich für eine Verbindung mit einem Nadelhalter für eine Injektionsnadel aufweist. Das Reservoirteil 1 dient der Aufnahme eines Reservoirbehältnisses, das im Ausführungsbeispiel von einer Ampulle 2 gebildet wird, die in dem Längsschnitt der Figur 3 erkennbar ist. Ein Auslass an dem vorderen Ende der Ampulle 2 wird von einer Membran fluiddicht verschlossen. Bei der Befestigung des Nadelhalters an dem vorderen Ende des Reservoirteils 1 durchsticht ein rückwärtiger Teil der Injektionsnadel die Membran, so dass eine Fluidverbindung zwischen der Spitze der hohlen Injektionsnadel und dem Reservoir 2 hergestellt ist.

Figur 3 zeigt das Injektionsgerät in seiner Gesamtheit in einem Längsschnitt. In der Ampulle 2 ist ein Kolben in die Vorschubrichtung auf den am vorderen Ende der Ampulle 2 gebildeten Auslass zu verschiebbar aufgenommen. Durch die Verschiebung des Kolbens in Vorschubrichtung wird Produkt aus der Ampulle 2 verdrängt und durch den Auslass und die Injektionsnadel hindurch ausgeschüttet.

Den Vorschub des Kolbens bewirkt die Kolbenstange 4, die mit ihrem vorderen Ende gegen den Kolben drückt und dadurch bei ihrem eigenen Vorschub den Kolben in die Vorschubrichtung bewegt. Die Kolbenstange 4 wird von dem Mechanikhalter 3 so gehalten, dass sie nach Überwindung eines gewissen Widerstands in die Vorschubrichtung, nicht jedoch entgegen der Vorschubrichtung bewegt werden kann. Die Rückwärtsbewegung der Kolbenstange 4 entgegen der Vorschubrichtung wird von einer Blockiereinrichtung 8 verhindert. Die Blockiereinrichtung 8 wird von dem Mechanikhalter 3 axial fixiert, d.h. sie ist in dem Mechanikhalter 3 so gehalten, dass sie in und gegen die Vorschubrichtung nicht bewegbar ist. Allerdings wird sie von dem Mechanikhalter 3 um die Längsachse L drehbar gelagert. Die Blockiereinrichtung 8 erzeugt auch den Widerstand, der für die Vorwärtsbewegung überwunden werden muss.

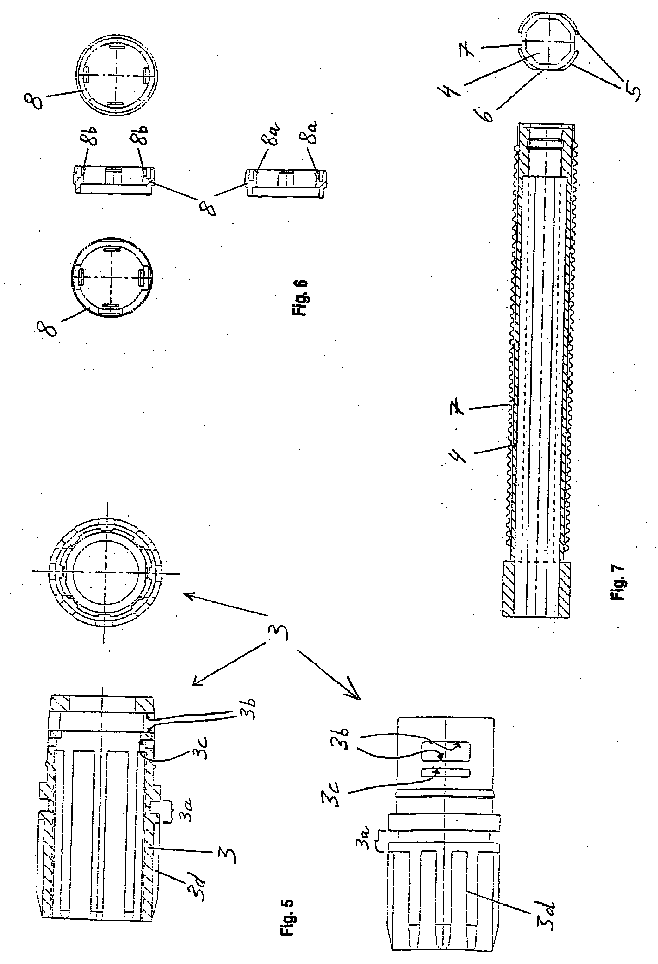

Die Blockiereinrichtung 8 ist in Figur 6 alleine dargestellt. Sie wird von einem einteiligen Ringelement gebildet, das an dem Mechanikhalter 3 zwischen zwei einander zugewandt beabstandeten Schultern 3b, die von einem Innenmantel des Mechanikhaltes 3 radial nach innen vorstehen, um die Längsachse L drehbar anliegt. Die Schultern 3b bilden eine Fixiereinrichtung für die axiale Fixierung der Blockiereinrichtung 8. Die Lagerung der Blockiereinrichtung 8 in dem Mechanikhalter 3 ist am besten aus der Darstellung des Mechanikhalters 3 in Figur 5 ersichtlich.

In dem Mechanikhalter 3 ist ferner ein Dosiseinstellglied 9 aufgenommen. Das Dosiseinstellglied 9 ist als Gewindemutter ausgebildet und steht mit einem Außengewinde der Kolbenstange 4 in einem Gewindeeingriff. Das Dosiseinstellglied 9 wird von dem Mechanikhalter 3 verdrehgesichert, aber in und gegen die Vorschubrichtung axial linear bewegbar geführt. Die Kolbenstange 4 und das Dosiseinstellglied 9 bilden einen Spindeltrieb, um die zu verabreichende Produktdosis auszuwählen.

Der Ampullenhalter 1 und der Mechanikhalter 3 sind verdreh- und verschiebegesichert miteinander verbunden und bilden zusammen das Reservoirmodul 10 des Injektionsgeräts, wobei dieses Reservoirmodul 10 die von dem Mechanikhalter 3 mittels der Blockiereinrichtung 8 gehaltene Kolbenstange 4 und das Dosiseinstellglied 9 umfasst. Der Ampullenhalter 1 und der Mechanikhalter 3 bilden zusammen einen vorderen Gehäuseabschnitt des Injektionsgeräts. Mit diesem vorderen Gehäuseabschnitt 1, 3 ist ein hinterer Gehäuseabschnitt 11 formschlüssig verbunden. Der hintere Gehäuseabschnitt 11 bildet den Träger eines Dosier- und Betätigungselements 12 und zusammen mit demselben sowie Teilen einer Verriegelungseinrichtung und weiteren Teilen ein Dosier- und Betätigungsmodul 30 des Injektionsgeräts.

Eine Dosier- und Betätigungsvorrichtung umfasst mit Ausnahme des Dosiseinstellglieds 9, der Kolbenstange 4 und der Blockiereinrichtung 8 die anderen Komponenten für die Auswahl der Produktdosis und die Betätigung des Injektionsgeräts. Insbesondere umfasst sie das Dosier- und Betätigungselement 12. Ferner umfasst die Dosier- und Betätigungsvorrichtung eine Zähl- und Anzeigeeinrichtung 17 zum Zählen und optischen Anzeigen der ausgewählten Produktdosis. Nicht zuletzt die Zähl- und Anzeigeeinrichtung 17 macht das Dosier- und Betätigungsmodul 30 zu einem hochwertigen und daher hochpreisigen Teil des Injektionsgeräts. Während das vergleichsweise preiswerte Reservoirmodul 10 als Einwegmodul konzipiert ist, ist das Dosier- und Betätigungsmodul 30 für die mehrmalige Verwendung mit immer wieder neuen Reservoirmodulen 10 bestimmt.

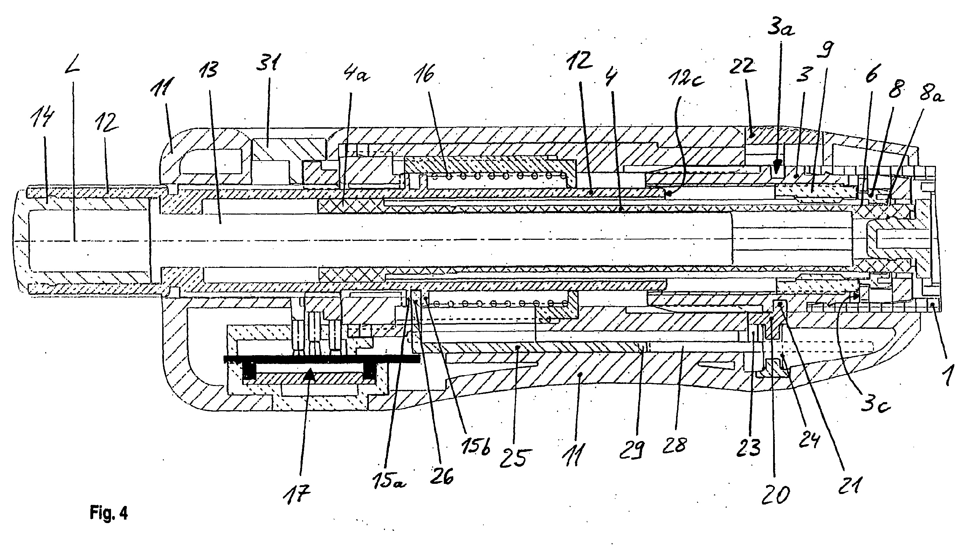

Für die Auswahl der Produktdosis, d.h. für die Dosierung, wird das Dosier- und Betätigungselement 12 um die Längsachse L drehbar und ferner in und gegen die Vorschubrichtung entlang der Längsachse L geradverschiebbar von dem hinteren Gehäuseabschnitt 11 gelagert. Das Dosier- und Betätigungselement 12 ist hohlzylindrisch und umgibt mit einem vorderen Abschnitt die Kolbenstange 4. Ein hinterer Abschnitt des Dosier- und Betätigungselements 12 ragt über ein hinteres Ende des Gehäuseabschnitts 11 hinaus. In das Dosier- und Betätigungselement 12 ist von hinten ein stangenförmiger Dosiermitnehmer 13 bis gegen eine radial nach innen vorragende Schulter des Dosier- und Betätigungselements 12 eingeschoben. Ferner ist an dem hinteren Ende ein Verschluss 14 bis gegen den Dosiermitnehmer 13 in das Dosier- und Betätigungselement 12 eingeschoben. Der Dosiermitnehmer 13 wird zwischen der radial vorragenden Schulter des Dosier- und Betätigungselements 12 und dem Verschluss 14 relativ zu dem Dosier- und Betätigungselement 12 axial fixiert. Der Dosiermitnehmer 13 ist mit dem Dosier- und Betätigungselement 12 außerdem verdrehgesichert verbunden. Der Dosiermitnehmer 13 ragt zum Zwecke der Dosierung von hinten in die hohle Kolbenstange 4 hinein. Die Kolbenstange 4 weist einen Verbindungabschnitt 4a auf (Fig. 4), der mit dem Dosiermitnehmer 13 in solch einem Eingriff ist, dass die Kolbenstande 4 und der Dosiermitnehmer 13 und mit diesem auch das Dosier- und Betätigungselement 12 relativ zueinander um die gemeinsame Längsachse L nicht verdrehbar, aber entlang der Längsachse L in und gegen die Vorschubrichtung relativ zueinander bewegbar sind. Hierfür ist der Verbindungsabschnitt 4a als Linearführung für den Dosiermitnehmer 13 ausgebildet.

Eine Rückstelleinrichtung 16 spannt das Dosier- und Betätigungselement 12 elastisch gegen die Vorschubrichtung in die in den Figuren 3 und 4 dargestellte Ausgangsstellung. In der Ausgangsstellung kann durch Drehung des Dosier- und Betätigungselements 12 um die Längsachse L die Dosierung vorgenommen werden. Anschließend kann aus der Ausgangsstellung heraus durch Axialverschiebung des Dosier- und Betätigungselements 12 die Ausschüttung der ausgewählten Produktdosis bewirkt werden. Die Rückstelleinrichtung 16 wird von einer als Druckfeder wirkenden Spiralfeder gebildet, die in einem Ringspalt um das Dosier- und Betätigungselement 12 aufgenommen ist und zwischen einer radial nach innen ragenden Schulter des Gehäuseabschnitts 11 und einer gegenüberliegend zugewandt radial nach außen ragenden Schulter des Dosier- und Betätigungselements 12 axial abgestützt ist.

Die Blockiereinrichtung 8 erfüllt eine Doppelfunktion. Zum einen stellt sie mit ihren Sperrelementen 8a sicher, dass die Kolbenstange 4 relativ zu dem Mechanikhalter 3 und damit insbesondere relativ zu dem in der Ampulle 2 aufgenommenen Kolben nicht entgegen der Vorschubrichtung zurückbewegt werden kann. In ihrer Doppelfunktion verhindert die Blockiereinrichtung 8 ferner als Bremse eine Vorwärtsbewegung der Kolbenstange 4 bei dem Dosiervorgang, bei dem das Dosiseinstellglied 9 entgegen der Vorschubrichtung axial auf das Dosier- und Betätigungselement 12 zu bewegt wird.

In der in den Figuren 3 und 4 dargestellten Ausgangsstellung vor einer Dosierung liegt das Dosiseinstellglied 9 in Vorschubrichtung auf Anschlag gegen einen von dem Mechanikhalter 3 gebildeten Ausschüttanschlag 3c (Figur 5). Die Kolbenstange 4 steht in einem permanenten Berührungskontakt mit dem Kolben. Zum Zwecke der Dosierung wird das Dosiseinstellglied 9 durch den Gewindeeingriff mit der Kolbenstange 4 und die Linearführung durch den Mechanikhalter 3 von dem Ausschüttanschlag 3c weg auf das Dosier- und Betätigungselement 12 zu bewegt. Hierdurch wird ein lichter Abstand zwischen einer rückwärtigen Anschlagfläche des Dosiseinstellglieds 9 und einer vorderen Anschlagfläche des Dosier- und Betätigungselements 12 verringert, andererseits jedoch der lichte Abstand zwischen einer vorderen Anschlagfläche des Dosiseinstellglieds 9 und dem Ausschüttanschlag 3c vergrößert. Der letztgenannte Abstand zwischen dem Ausschüttanschlag 3c und dem Dosiseinstellglied 9 ist die Weglänge, um die im Zuge der Ausschüttbewegung des Dosier- und Betätigungselements 12 das Dosiseinstellglied 9 und wegen des Gewindeeingriffs auch die Kolbenstange 4 in die Vorschubrichtung bewegt werden. Der Ausschüttanschlag 3c bildet einen vorderen Translationsanschlag. Bei der Ausschüttbewegung drückt die Kolbenstange 4 mit ihrem vorderen Ende, das von einem mit der Kolbenstange 4 in und gegen die Vorschubrichtung nicht bewegbar verbundenen Stempelkörper gebildet wird, gegen den Kolben und drückt den Kolben in die Vorschubrichtung auf den Auslass der Ampulle 2 zu vor. Die Längsachse L bildet die Rotations- und Translationsachse der Bewegungen, die zum Zwecke der Dosierung und Produktausschüttung ausgeführt werden.

Der Abstand, den das Dosiseinstellglied 9 und das Dosier- und Betätigungselement 12 vor dem Dosiervorgang zwischen sich aufweisen, wenn das Dosiseinstellglied 9 auf Anschlag gegen den Ausschüttanschlag 3c liegt, entspricht der maximalen Produktdosis, die ausgewählt und im Zuge einer Ausschüttung ausgeschüttet werden kann. Die Hubbewegung des Dosier- und Betätigungselements 12 ist bei jeder Ausschüttung gleich lang. Es wird durch die Dosierung lediglich der Abstand des Dosiseinstellglieds 9 von dem Ausschüttanschlag 3c und damit die im Zuge der Ausschüttung von dem Dosier- und Betätigungselement 12 und dem Dosiseinstellglied 9 gemeinsam zurücklegbare Weglänge eingestellt.

Die Bremsfunktion der Blockiereinrichtung 8 und der hierfür zwischen der Kolbenstange 4 und der Blockiereinrichtung 8 bestehende Bremseingriff werden aus der Zusammenschau der Figuren 6 und 7 deutlich. Zum einen weist die Blockiereinrichtung 8 für den Bremseingriff zwei Bremselemente 8b auf, die, wie bereits die Sperrelemente 8a, je von einem elastisch nachgebenden Schnapper gebildet werden. Die Blockiereinrichtung 8 wird im Ausführungsbeispiel von einem einzigen Ringelement gebildet, von dem an einer Stirnseite vier elastische Schnapper axial abragen. Die Schnapper sind gleichmäßig über den Umfang des Ringelements verteilt angeordnet. Zwei einander gegenüberliegende Schnapper bilden die Sperrelemente 8a und die zwei weiteren, einander ebenfalls gegenüberliegend angeordneten Schnapper bilden die Bremselemente 8b.

Die Kolbenstange 4 weist dementsprechend zwei am Außenmantel an gegenüberliegenden Seiten ausgebildete und in Längsrichtung der Kolbenstange 4 sich erstreckende Rückzugsperreinrichtungen 6 und zwei an ebenfalls einander gegenüberliegenden Seiten in Längsrichtung der Kolbenstange 4 sich erstreckende Vorschubbremseinrichtungen 7 auf. Das Gewinde der Kolbenstange 4 für den Gewindeeingriff mit dem Dosiseinstellglied 9 wird von vier verbleibenden Gewindeabschnitten 5 gebildet, die sich über nahezu die gesamte Länge der Kolbenstange 4 erstrecken. Die Rückzugsperreinrichtungen 6 und die Vorschubbremseinrichtungen 7 werden je von einer Zahnreihe gebildet. Während die Zähne der Rückzugsperreinrichtungen 6 jedoch als in Vorschubrichtung gepfeilte Sägezähne mit nach hinten weisenden, quer zur Vorschubrichtung sich erstreckenden Sperrflächen gebildet sind, weisen die beiden Zahnreihen, die die Vorschubbremseinrichtungen 7 bilden, nach vorne weisende Sperrflächen mit einer vergleichbaren Sperrwirkung nicht auf. Die Zähne der Vorschubbremseinrichtungen 7 weisen je ein im Vergleich zu den Rückzugssperreinrichtungen 6 weicheres Zahnprofil auf. Der Bremseingriff der Blockiereinrichtung 8 mit den Vorschubbremseinrichtungen 7 der Kolbenstange 4 soll eine Vorschubbewegung der Kolbenstange 4 nämlich nicht verhindern, sondern nur erschweren, um sicherzustellen, dass die Kolbenstange 4 nicht bei der Dosierung in Vorschubrichtung bewegt wird. Die Zähne der Vorschubbremseinrichtungen 7 sind an ihren Vorderseiten und die Bremselemente 8b sind an ihren Rückseiten, die die Vorderseiten der Zähne der Vorschubbremseinrichtung 7 berühren, so geformt, dass zur Überwindung des Bremseingriffs eine Schwellkraft überwunden werden muss, die bei der Dosierung nicht erreicht wird. Diese Schwellkraft ist größer als die Kraft, die erforderlich ist, um die Zähne der Rückzugssperreinrichtungen 6 über die Sperrelemente 8a in Vorschubrichtung zu bewegen. Vorzugsweise ist die Schwellkraft wenigstens doppelt so groß wie die anfängliche Reibkraft zwischen den Rückzugssperreinrichtungen 6 und den Sperrelementen 8a. Die Reibkraft zwischen letzteren vergrößert sich auch erst im Verlaufe der Vorschubbewegung allmählich zwischen zwei aufeinanderfolgenden Sperreingriffen. Die Schwellkraft des Bremseingriffs muss hingegen in jedem Sperreingriff unmittelbar am Beginn der Vorschubbewegung von einem Sperreingriff in den nächst folgenden aufgebracht werden. Allerdings soll die Schwellkraft nicht so groß sein, dass sie vom Verwender bei der Ausschüttung als störend empfunden wird.

Eine unerwünschte Vorschubbewegung der Kolbenstange als Reaktion auf die Bewegung des Dosiseinstellglieds 9 bei der Dosisauswahl kann grundsätzlich auch allein durch den Sperreingriff der Blockiereinrichtung 8 bewirkt werden. Allerdings wird solch eine Bewegung wegen des Bremseingriffs sicherer verhindert als durch den Sperreingriff allein.

Die Verbindung zwischen dem Reservoirmodul 10 und dem Dosier- und Betätigungsmodul 30 ist formschlüssig. Zum einen besteht zwischen dem Mechanikhalter 3 und dem Gehäuseabschnitt 11 ein Verriegelungseingriff, der eine Relativbewegung in axialer Richtung verhindert. Über den Verriegelungseingriff hinaus sind der vordere Gehäuseabschnitt 1, 3 und der hintere Gehäuseabschnitt 11 unmittelbar aneinander axial linear geführt, um eine Relativdrehung bei dem Verbinden und im verbundenem Zustand zu verhindern. In Figur 5 sind die Axialführungen 3d des Mechanikhalters 3, die mit einem oder mehreren entsprechenden Eingriffselementen des hinteren Gehäuseabschnitts 11 die Linearführung bilden, gut zu erkennen. Die Axialführungen 3d werden von Führungsflächen an Führungsrippen gebildet; sie könnten auch von Führungsflächen in axial sich erstreckenden Vertiefungen gebildet werden. Auf diese Weise werden axiale Führungskanäle erhalten. Die Führungsrippen sind axial verjüngt, so dass in die Führungskanäle führende Einführtrichter für das eine oder die mehreren Eingriffselemente des hinteren Gehäuseabschnitts 11 gebildet werden. Zur noch besseren Zentrierung der Gehäuseabschnitte 1, 3 und 11 zu Beginn des Verbindens sind die Führungsrippen auch noch in radialer Richtung verjüngt. Das eine oder die mehreren Eingriffselemente des hinteren Gehäuseabschnitts 11 ist oder sind vorzugsweise wie die Axialabschnitte 3d an der Mantelgegenfläche, d.h. der Innenmantelfläche, des hinteren Gehäuseabschnitts 11 gebildet.
Der Verriegelungseingriff besteht zwischen einem weiblichen, ersten Verriegelungselement 3a des Mechanikhalters 3 (Figur 5) und einem Verriegelungsring 20, der mit dem hinteren Gehäuseabschnitt 11 radial bewegbar, aber axial nicht bewegbar verbunden ist. Der Verriegelungsring 20 bildet ein unmittelbar in das erste Verriegelungselement 3a radial eingreifendes männliches, zweites Verriegelungselement 21. Zwischen dem ersten Verriegelungselement 3a und dem zweiten Verriegelungselement 21 besteht eine Schloss/Riegel-Verbindung, die axiale Relativbewegungen zwischen dem Reservoirmodul 10 und dem Dosier- und Betätigungsmodul 30 verhindert.

Die Figuren 3 und 4 zeigen das Verriegelungselement 21 im Verriegelungseingriff mit dem Verriegelungselement 3a. Das Verriegelungselement 3a wird von einem Ringsteg und einer Nut gebildet, die an dem Außenmantel des Mechanikhalters 3 umlaufen. Der Ringsteg bildet eine hintere Seitenwand der Nut. Das zweite Verriegelungselement 21 wird von einem Nocken gebildet, der von dem Innenmantel des Verriegelungsrings 20 radial einwärts vorragt und im Verriegelungseingriff von einer Rückstelleinrichtung 24 radial einwärts über eine Innenmantelfläche des hinteren Gehäuseabschnitts 11 vorstehend in das aufnehmende Verriegelungselement 3a hineingedrückt wird. Der Verriegelungsring 20 ist in seiner Gesamtheit in Radialrichtung mittels der Rückstelleinrichtung 24 an einer von dem Gehäuseabschnitt 11 gebildeten Innenmantelfläche abgestützt, so dass die Rückstelleinrichtung 24 etwa in radialer Verlängerung des Verriegelungselements 21 gegen den Außenmantel des Verriegelungsrings 20 drückt. Der Verriegelungsring 20 umgibt den Mechanikhalter 3 und ist in seiner Gesamtheit gegen die rückstellende Kraft der Rückstelleinrichtung 24 radial hin und her bewegbar, so dass das zweite Verriegelungselement 21 in und aus dem Verriegelungseingriff mit dem ersten Verriegelungselement 3a bringbar ist. Der hintere Gehäuseabschnitt 11 bildet eine enge Gleitführung für die Radialbewegung des Verriegelungsrings 20. An seiner dem Verriegelungselement 21 radial gegenüberliegenden Seite bildet der Verriegelungsring 20 einen Entriegelungsknopf 22 für den Verwender. Zur radialen Führung der als Druckfeder ausgebildeten Rückstelleinrichtung 24 ragt von der dem Verriegelungselement 21 abgewandten Außenmantelfläche des Verriegelungsrings 20 ein Führungsnocken radial ab.

In Umfangsrichtung zu beiden Seiten dieses Führungsnockens und axial hinter dem Führungsnocken ragen von der Außenmantelfläche des Verriegelungsrings 20 ferner zwei Sperrnocken 23 ab, die in Radialrichtung nach außen gegen eine Verriegelungssperre 25 drücken. Aufgrund der Anlage der Sperrnocken 23 gegen die Verriegelungssperre 25 wird eine Radialbewegung des Verriegelungselements 21 verhindert, die zu einem Lösen des Verriegelungseingriffs führen könnte. Der zwischen den Verriegelungselementen 3a und 21 bestehende Verriegelungseingriff wird somit durch die Verriegelungssperre 25 gesichert. Diese Sicherung besteht in jeder Verschiebeposition des Dosier- und Betätigungselements 12 mit Ausnahme einer Freigabestellung, die das Dosier- und Betätigungselement 12 am Ende seiner Ausschüttbewegung einnimmt. Die Freigabestellung fällt daher mit der vordersten Verschiebeposition zusammen, die das Dosier- und Betätigungselement 12 dann einnimmt, wenn es im Zuge seiner Ausschüttbewegung an das Dosiseinstellglied 9 und das Dosiseinstellglied 9 seinerseits gegen den Ausschüttanschlag 3c des Mechanikhalters 3 anstößt. Solange das Dosier- und Betätigungsmodul 30 mit dem Reservoirmodul noch nicht verbunden ist, wird ein mechanischer Anschlag für das Dosier- und Betätigungselement 12 von einem Anschlagelement 31 der Dosier- und Betätigungsvorrichtung gebildet. Im Ausführungsbeispiel bildet ein Resethalterring, der einem Reset der Anzeige 17 dient, das Anschlagelement 31. Der Anschlag des Dosier- und Betätigungselement 12 gegen dieses Anschlagelement 31 definiert die Freigabestellung des Dosier- und Betätigungselements 12 in diesem Falle, wobei die durch das Anschlagelement 31 definierte Freigabestellung derjenigen entspricht, die durch das Anstoßen des Dosiseinstellglieds 9 an dem Ausschüttanschlag 3c definiert wird.

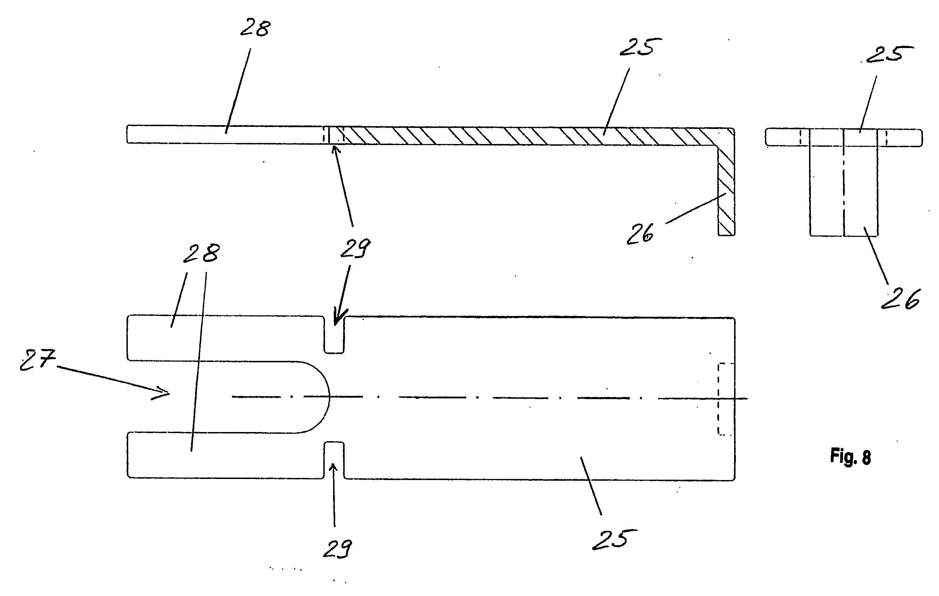

Figur 8 zeigt die Verriegelungssperre 25. Sie wird im Ausführungsbeispiel einstückig von einem Sperrschieber gebildet. Die Verriegelungssperre 25 weist einen plattenförmigen Hauptkörper auf, der sich im montierten Zustand, wie beispielsweise in Figur 4 dargestellt, axial erstreckt. An einem Ende ragt von dem Hauptkörper ein Steg 26 rechtwinklig ab. Im montierten Zustand erstreckt sich der Steg 26 radial bis gegen das Dosier- und Betätigungselement 12. Der Steg 26 dient der Befestigung der Verriegelungssperre 25 an dem Dosier- und Betätigungselement 12, das hierfür zwei axial beabstandet an einer Außenmantelfläche gebildete Ringstege aufweist, die Mitnehmer 15a und 15b bilden. Der vordere Mitnehmer 15b bildet gleichzeitig die Stützschulter für die Rückstelleinrichtung 16. In den zwischen den Mitnehmern 15a und 15b gebildeten Ringraum ragt die Verriegelungssperre 25 mit ihrem Steg 26 ein und wird von den beiden Mitnehmern 15a und 15b axial beidseits eng eingefasst.

An einem von dem Steg 26 abgewandten vorderen Ende ist der Hauptkörper der Verriegelungssperre 25 mit einer Axialausnehmung 27 versehen, die zu dem vorderen Ende der Verriegelungssperre 25 hin offen ist. Auf diese Weise werden beidseits der Ausnehmung 27 axial sich erstreckende Sperrzungen 28 gebildet. Die beiden Sperrnocken 23 des Verriegelungsrings 20 sind so angeordnet, dass je einer dieser Sperrnocken 23 gegen eine der Sperrzungen 28 drückt, solange das Dosier- und Betätigungselement 12 nicht die Freigabestellung einnimmt. Durch die axiale Ausnehmung 27 hindurch erstreckt sich bei der Axialbewegung der Verriegelungssperre 25 die Rückstelleinrichtung 24 für das Verriegelungselement 21.

In dem Hauptkörper der Verriegelungssperre 25 sind ferner Einrückausnehmungen 29 gebildet, die die Freigabestellung des Dosier- und Betätigungselements 12 definieren. Pro Sperrnocken 23 ist je eine Einrückausnehmung 29 vorgesehen. Die Position der Einrückausnehmungen 29 ist so gewählt, dass sie mit den Sperrnocken 23 erst dann in Überdeckung gelangen und dadurch ein Einfahren der Sperrnocken 23 gestatten, wenn das Dosier- und Betätigungselement 12 bis in seine Freigabestellung vorbewegt worden ist.

Es ist klar, dass bei der im Ausführungsbeispiel speziell gewählten Anordnung auch ein einziger Sperrnocken 23 vorgesehen sein könnte und die Verriegelungssperre 25 dementsprechend auch nur eine einzige Einrückausnehmung 29 und gegebenenfalls auch nur eine Sperrzunge 28 aufweisen könnte. Grundsätzlich könnte die Verriegelungssperre ferner einstückig mit dem Dosier- und Betätigungselement 12 gefertigt sein. Die Ausbildung als separates Teil bietet jedoch Vorteile hinsichtlich der Fertigung, der Montage und dem Zusammenwirken des Dosier- und Betätigungselements 12 mit der Kolbenstange 4. In Bezug auf die Einbaulage der Verriegelungssperre 25 ist noch darauf hinzuweisen, dass die Verriegelungssperre 25 an ihrer von dem Verriegelungselement 21 abgewandten Außenseite an einer Innenmantelfläche des Gehäuses 11 abgestützt ist. Auf diese Weise wird die Stabilität der Sicherung für den Verriegelungseingriff erhöht. Vorzugsweise bildet das Gehäuse 11 eine Axialführung für die Verriegelungssperre 25.

Im Folgenden wird die Funktionsweise des Injektionsgeräts beschrieben, wobei angenommen sei, dass ein neues Reservoirmodul 10 und ein bereits wenigstens einmal verwendetes Dosier- und Betätigungsmodul 30 zusammengebaut und anschließend eine erste Produktausschüttung vorgenommen werden.

Das Dosier- und Betätigungsmodul 30 und das neue Reservoirmodul 10 werden axial zueinander ausgerichtet, so dass ihre beiden Längsachsen miteinander fluchten. Anschließend wird das Reservoirmodul 10 mit seinem rückwärtigen Ende in das nach vorne offene Gehäuse 11 des Dosier- und Betätigungsmoduls 30 eingeführt.

Hierbei zentrieren sich der Gehäuseabschnitt 1, 3 und der Gehäuseabschnitt 11 an den verjüngten Enden der Führungsrippen 3d des Mechanikhalters 3. Während des Aufschiebens werden die beiden Gehäuseabschnitte in einer durch die Linearführung vorgegebenen Drehwinkelposition axial aneinander lineargeführt, bis die Gehäuseabschnitte 1, 3 und 11 eine Verbindungsendposition einnehmen, in der der Verriegelungseingriff der Verriegelungselemente 3a und 21 hergestellt werden kann oder von selbst sich einstellt.

Das Dosier- und Betätigungselement 12 ist in vorgegebenen Drehwinkelpositionen relativ zu dem hinteren Gehäuseabschnitt 11 gerastet. Die Linearführung der Gehäuseabschnitte 1, 3 und 11 und die Drehwinkelrastposition des Dosier- und Betätigungselements 12 sind so aufeinander abgestimmt, dass der verdrehgesicherte Eingriff des Dosier- und Betätigungselements 12 mit der Kolbenstange 4 in jeder Rastposition des Dosier- und Betätigungselements 12 und jeder Drehwinkelposition, in der die Gehäuseabschnitte 1, 3 und 11 aneinander lineargeführt sind, hergestellt wird.

Falls das Dosier- und Betätigungselement 12 sich in einer Axialposition relativ zu dem Gehäuseabschnitt 11 befindet, die hinter der Freigabestellung liegt, wird das Verriegelungselement 21 von der Verriegelungssperre 25 in seiner radial innersten Stellung gehalten. In dieser Stellung des Verriegelungselements 21 können das Dosier- und Betätigungsmodul 30 und das Reservoirmodul 10 nicht bis in die Verbindungsendstellung aufeinandergeschoben und deshalb auch nicht miteinander verbunden werden, da der am Außenmantel des Mechanikhalters 3 gebildete Ringsteg, der das erste Verriegelungselement 3a mitbildet, vorher gegen das zweite Verriegelungselement 21 auf Anschlag zu liegen kommt.

Der Ringsteg kann zu einem in tangentialer Richtung kurzen Radialvorsprung reduziert werden, wenn dafür gesorgt ist, dass die Gehäuseabschnitte 1, 3 und 11 nur in der Drehwinkellage zusammengebaut werden können, in der solch ein Vorsprung und das zweite Verriegelungselement 21 in einer axialen Flucht zu liegen kommen. Der Ringsteg oder Radialvorsprung könnte das erste Verriegelungselement 3a auch alleine bilden, da es die wesentliche Funktion des ersten Verriegelungselements 3a ist, die Herstellung der Verbindung zwischen dem Reservoirmodul 10 und dem Dosier- und Betätigungsmodul 30 nur zuzulassen, wenn das Dosier- und Betätigungselement 12 seine Freigabestellung einnimmt. Ist diese Bedingung erfüllt, so würde das Dosier- und Betätigungselement 12 bei der Herstellung der Verbindung zwischen dem Reservoirmodul 10 und dem Dosier- und Betätigungsmodul 30 sicherstellen, dass das Dosiseinstellglied 9 sich in seiner Dosiernullstellung befindet, in der es an dem Ausschüttanschlag 3c des Mechanikhalters 3 anstößt.

Um den die vorstehend erläuterte Bedingung erfüllenden Zustand herzustellen, drückt der Verwender das Dosier- und Betätigungselement 12 axial relativ zu dem hinteren Gehäuseabschnitt 11 bis in die Freigabestellung vor. In dieser Relativstellung zwischen Gehäuseabschnitt 11 1 und Dosier- und Betätigungselement 12 können die Sperrnocken 23 in die Einrückaufnahmen 29 der Verriegelungssperre 25 bewegt werden. Der Verwender drückt daher nicht nur das Dosier- und Betätigungselement 12 bis wenigstens in die Freigabestellung vor, sondern gleichzeitig auch mittels des Entriegelungsknopfs 22 das erste Verriegelungselement 20 aus dem Verriegelungseingriff. Das Reservoirmodul 10 kann nun axial über den Ringsteg des ersten Verriegelungselements 3a bewegt und weiter in den hinteren Gehäuseabschnitt 11 eingeführt werden. Der Verwender kann den Entriegelungsknopf 22 loslassen. Sobald das erste Verriegelungselement 21 mit dem zweiten Verriegelungselement 3a in Überdeckung gelangt, schnappt es aufgrund der Kraft der Rückstelleinrichtung 24 in das aufnehmende Verriegelungselement 3a ein, so dass der Verriegelungseingriff hergestellt ist. Das Reservoirmodul 10 und das Dosier- und Betätigungsmodul 30 sind nun in Bezug auf die Stellung des Dosiseinstellglieds 9 und der Kolbenstange 4 in definierter Weise miteinander verbunden. Falls das Dosiseinstellglied 9 vor Herstellung des Verriegelungseingriffs noch einen lichten Abstand von dem Ausschüttanschlag 3c aufgewiesen hat, ist dieser Abstand aufgrund der zum Herstellen der Verbindung erforderlichen Einwirkung des Dosier- und Betätigungselements 12 beseitigt. Eine damit einhergehende Produktausschüttung kann hingenommen werden und kann zum Zwecke des Primens der Injektionsnadel sogar erwünscht sein. Die Zähl- und Anzeigeeinrichtung 17 wird vorzugsweise dabei genullt.

In dem derart herbeigeführten, definierten Ausgangszustand kann der Verwender die Dosierung vornehmen. Die Dosierung erfolgt durch Drehung des Dosier- und Betätigungselements 12 um die Längsachse L und relativ zu dem Gehäuseabschnitt 11. Da der Dosiermitnehmer 13 mit dem Dosier- und Betätigungselement 12 verdrehgesichert verbunden ist und seinerseits verdrehgesichert in die Kolbenstange 4 eingreift, nimmt das Dosier- und Betätigungselement 12 bei der rotatorischen Dosierbewegung die Kolbenstange 4 mit. Aufgrund des Gewindeeingriffs zwischen der Kolbenstange 4 und dem Dosiseinstellglied 9 und der Linearführung des Dosiseinstellglieds 9 durch den Mechanikhalter 3 führt das Dosiseinstellglied 9 eine von der Gewindesteigung des gegenseitigen Gewindeeingriffs vorgegebene, axiale translatorische Dosierbewegung in Richtung auf das Dosier- und Betätigungselement 12 aus. Das Dosier- und Betätigungselement 12 bildet einen hinteren Translationsanschlag 12c, der die translatorische Dosierbewegung des Dosiseinstellglieds 9 begrenzt und dadurch den maximal einstellbaren Ausschütthub definiert.

Die Zähl- und Anzeigeeinrichtung 17 zählt die der Drehwinkelstellung des Dosier- und Betätigungselements 12 entsprechenden Dosiseinheiten und zeigt sie optisch an.

Nach Auswahl der gewünschten Produktdosis ist der Dosiervorgang beendet. Die Ausschüttung der gewählten Produktdosis wird mittels der in Vorschubrichtung des Kolbens weisenden Ausschüttbewegung des Dosier- und Betätigungselements 12 bewirkt. Im Zuge seiner Ausschüttbewegung stößt das Dosier- und Betätigungselement 12 gegen das Dosiseinstellglied 9 und nimmt es mit. Indem das Dosiseinstellglied 9 im Zuge der Ausschüttbewegung gegen den Ausschüttanschlag 3c des Mechanikhalters 3 stößt, werden die Ausschüttbewegungen des Dosier- und Betätigungselements 12 und die Produktausschüttung beendet. Nach dem Loslassen des Dosier- und Betätigungselements 12 vorzugsweise wird dieses von dem Rückstelleinrichtung 16 entgegen der Vorschubrichtung wieder in eine neue Ausgangsstellung für eine erneute Dosierung und Produktausschüttung bewegt. Die Zähl- und Anzeigeeinrichtung 17 ist mit dem Dosier- und Betätigungselement 12 vorzugsweise so gekoppelt, dass sie mittlerweile wieder auf Null zurückgestellt worden ist. Gegebenenfalls verfügt sie über Mittel zum Zählen und Anzeigen der bereits insgesamt ausgeschütteten Produktmenge und somit der in der Ampulle 2 verbliebenen Produktrestmenge.

Um das Reservoirmodul 10 von dem Dosier- und Betätigungsmodul 30 zu lösen, wird das Dosier- und Betätigungselement 12 bis in die Freigabestellung, d.h. bis auf Anschlag gegen das Dosiseinstellglied 9, vorbewegt. In dieser Stellung kann der Verwender durch Druck auf den Entriegelungsknopf 22 den Verriegelungseingriff wieder lösen und das Reservoirmodul 10 von dem Dosier- und Betätigungsmodul 30 trennen.

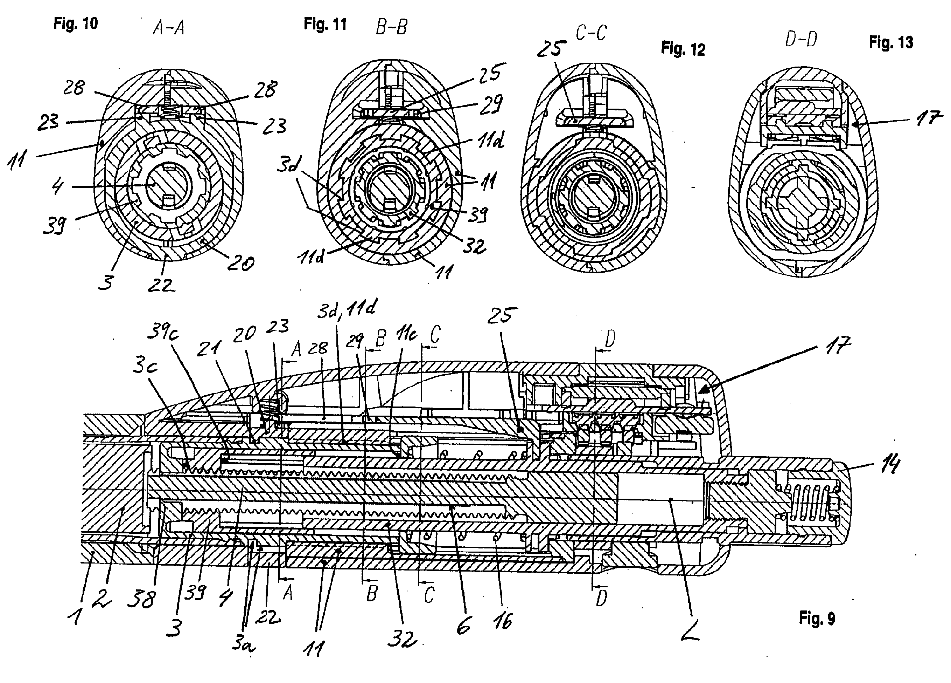

Die Figuren 9 bis 13 zeigen in einem Längsschnitt und vier Querschnitten ein zweites Ausführungsbeispiel eines Injektionsgeräts. Das Injektionsgerät des zweiten Ausführungsbeispiels gleicht demjenigen des ersten Ausführungsbeispiels in Bezug auf die Verriegelung und die Verriegelungssperre 25 derart, dass auf die diesbezügliche Beschreibung des ersten Ausführungsbeispiels verwiesen wird. Insbesondere ist die Verriegelungssperre 25 des zweiten Ausführungsbeispiels in Bezug auf alle funktionalen Details mit derjenigen des ersten Ausführungsbeispiels identisch. Das gleiche gilt für die Verriegelungselemente 3a und 21.

Der Verriegelungsring 20 und die Lage der Sperrnocken 23 relativ zu dem Verriegelungselement 21 und relativ zu der Verriegelungssperre 25 in dem Ausgangszustand des Geräts sind besonders gut in den Querschnitten der Figuren 10, 11 und 12 zu erkennen, auf die diesbezüglich auch stellvertretend für das erste Ausführungsbeispiel verwiesen wird.

Das Injektionsgerät des zweiten Ausführungsbeispiels unterscheidet sich von dem ersten Ausführungsbeispiel durch den Eingriff und den Bewegungsablauf der an der Dosierung beteiligten Komponenten. Ferner erfüllt der Mechanikhalter über die Funktionen des Mechanikhalters des ersten Ausführungsbeispiels hinaus insbesondere die Funktion der Positionierung des Dosiseinstellglieds in diskreten Drehwinkelpositionen, die relativ zu dem Mechanikhalter zum Zwecke der Dosierung veränderbar sind. Die Blockiereinrichtung des zweiten Ausführungsbeispiels ist hingegen einfacher ausgeführt als die des ersten Ausführungsbeispiels. Im Folgenden werden in erster Linie nur die Unterschiede im Vergleich zum ersten Ausführungsbeispiel beschrieben, wobei für Komponenten, die ihrer grundsätzlichen Funktion nach den gleichnamigen Komponenten des ersten Ausführungsbeispiels gleichen, aber in Details unterscheiden, 30iger Zahlen mit gleicher Endziffer oder exakt die gleichen Bezugszeichen, wie bei dem ersten Ausführungsbeispiel, verwendet werden. Soweit zum zweiten Ausführungsbeispiel keine Ausführungen gemacht werden, sollen die entsprechenden Ausführungen zum ersten Ausführungsbeispiel gelten.

In dem zweiten Ausführungsbeispiel ist das relativ zu dem hinteren Gehäuseabschnitt 11 axial linear bewegbare und um die Längsachse L verdrehbare Dosier- und Betätigungselement 32 mit dem Dosiseinstellglied 39 verdrehgesichert verbunden. Das Dosier- und Betätigungselement 32 und das Dosiseinstellglied 39 sind relativ zueinander und relativ zu den Gehäuseabschnitten 1, 3 und 11 in und gegen die Vorschubrichtung bewegbar. Die Kolbenstange 4 wird von dem Mechanikhalter 3 verdrehgesichert gehalten. Die mit dem ersten Ausführungsbeispiel funktionsgleiche Rückzugsperreinrichtung 6 verhindert im Zusammenwirken mit Sperrelementen der einstückig an dem Mechanikhalter 3 geformten Blockiereinrichtung 38 eine Bewegung der Kolbenstange 4 entgegen der Vorschubrichtung, lässt eine Bewegung in die Vorschubrichtung jedoch zu. Die Sperrelemente bilden gleichzeitig die Rückzugsperre und die Verdrehsicherung für die Kolbenstange 4. Des Weiteren bildet wie bereits im ersten Ausführungsbeispiel das Dosier- und Betätigungselement 32 eine Gleitführung für die Kolbenstange 4.

Bei der Dosierung führt das Dosier- und Betätigungselement 32 die gleiche rotatorische Dosierbewegung wie das Dosier- und Betätigungselement 12 des ersten Ausführungsbeispiels aus. Allerdings wird aufgrund des verdrehgesicherten Eingriffs das Dosiseinstellglied 39 bei der rotatorischen Dosierbewegung mitgenommen. Der Gewindeeingriff zwischen der Kolbenstange 4 und dem Dosiseinstellglied 39 ist wieder mit demjenigen des ersten Ausführungsbeispiels vergleichbar, so dass ein von dem Dosiseinstellglied 39 gebildeter Anschlag 39c aufgrund der rotatorischen Dosierbewegung und des Gewindeeingriffs mit der Kolbenstange 4 im Zuge der Dosierung entgegen der Vorschubrichtung auf ein vorderes Ende des Dosier- und Betätigungselements 32 zu bewegt wird. Im Gegensatz zum ersten Ausführungsbeispiel vollführt das Dosiseinstellglied 39 somit bei der Dosierung eine rotatorische Dosierbewegung und eine translatorische Dosierbewegung relativ zu dem vorderen Gehäuseabschnitt, während die Kolbenstange 4 stillsteht. Durch die nach Abschluss der Dosierung erfolgende Ausschüttbewegung des Dosier- und Betätigungselements 32 wird die Kolbenstange 4 um die Weglänge vorgeschoben, die dem durch die Dosierung eingestellten lichten Abstand zwischen einer Anschlagfläche des Dosiseinstellglieds 39 und dem Ausschüttanschlag 3c des Mechanikhalters 3 entspricht.

Die translatorische Dosierbewegung des Dosiseinstellglieds 39 wird gegen die Vorschubrichtung von einem hinteren Translationsanschlag 11 c begrenzt, den der hintere Gehäuseabschnitt 11 selbst unmittelbar bildet. Auch bei dem zweiten Ausführungsbeispiel bildet die Längsachse L die Rotations- und Translationsachse der Komponenten, die an der Dosierung und Produktausschüttung beteiligt sind.

Der vordere Gehäuseabschnitt 1, 3 bildet wie bei dem ersten Ausführungsbeispiel eine Gleitführung für das Dosiseinstellglied 39. Zur Ausbildung der Gleitführung stehen eine innere Mantelfläche des Mechanikhalters 3 und eine äußere Mantelfläche des Dosiseinstellglieds 39 miteinander in Gleitkontakt. Das Dosier- und Betätigungselement 32 steht mit einer Innenmantelfläche des Dosiseinstellglieds 39 in einem Eingriff, um die verdrehgesicherte Verbindung zwischen dem Dosiseinstellglied 39 und dem Dosier- und Betätigungselement 32 zu bilden.

In dem zweiten Ausführungsbeispiel weist die Kolbenstange 4 über die Rückzugssperreinrichtung 6 hinaus keine eigene Bremseinrichtung auf. Die Vorderseiten der Sägezähnen der Rückzugssperreinrichtung 6 bilden vielmehr alleine auch die Bremseinrichtung. Die Kolbenstange 4 des zweiten Ausführungsbeispiels kann jedoch durch die Kolbenstange des ersten Ausführungsbeispiels ersetzt werden. Entsprechend wären in diesem Falle durch den Mechanikhalter 3 des zweiten Ausführungsbeispiels auch wenigstens ein Bremselement, vorzugsweise beide Bremselemente des ersten Ausführungsbeispiels auszubilden.

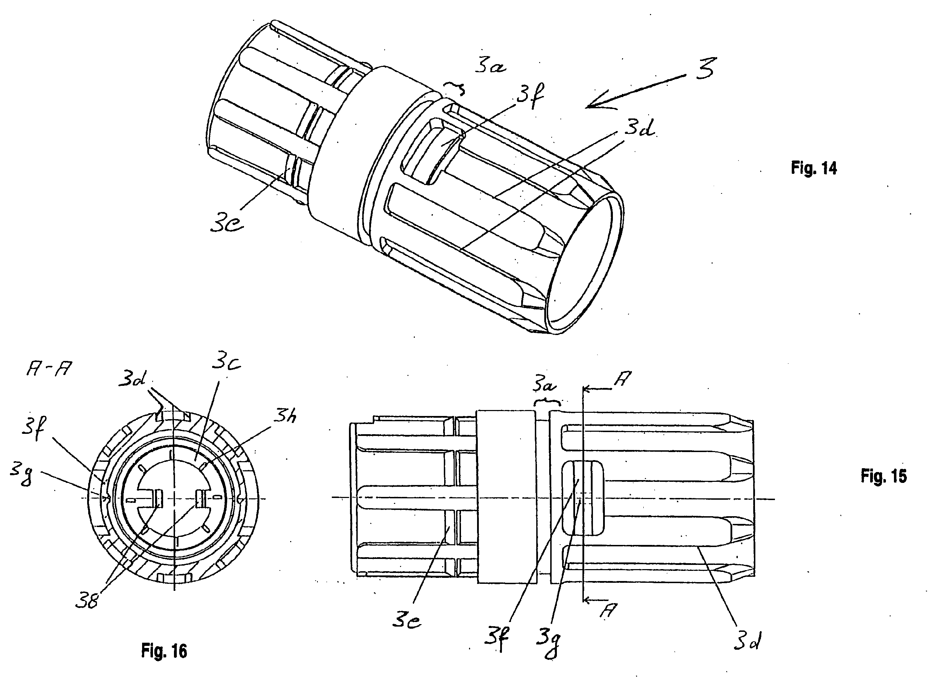

Die Figuren 14 bis 16 zeigen den Mechanikhalter 3 des zweiten Ausführungsbeispiels in einer perspektivischen Darstellung, einer Seitenansicht und in dem in der Seitenansicht eingetragenen Querschnitt A-A. Der Mechanikhalter 3 ist wie beim ersten Ausführungsbeispiel als einteiliges Hülsenteil ausgeführt, vorzugsweise als Kunststoffspritzgussteil. Er weist an dem Außenmantel eines vorderen Hülsenabschnitts einen Wulst 3e auf. Der vordere Hülsenabschnitt wird in das Reservoirteil 1 eingesteckt und mittels des Wulstes 3e zumindest für den Verwender unlösbar mit dem Reservoirteil 1 verrastet.

Das Verriegelungselement 3a ist an einem mittleren Hülsenabschnitt des Mechanikhalters 3 wie bei dem ersten Ausführungsbeispiel ausgebildet.

Ein hinterer Hülsenabschnitt, der sich an das Verriegelungselement 3a anschließt, bildet an seinem Außenumfang eine Mehrzahl von Axialführungen 3d. Die Axialführungen 3d werden von Führungsrippen gebildet, die an dem Außenumfang des hinteren Hülsenabschnitts radial aufragen. Genauer gesagt wird die Axialführung von den axial sich erstreckenden, geraden Seitenwänden dieser Führungsrippen gebildet, so dass, wie bei dem ersten Ausführungsbeispiel, axiale Führungskanäle erhalten werden. Die Führungsrippen ragen von dem mittleren Hülsenabschnitt aus wie Finger bis an das hintere Ende des Mechanikhalters 3, wo sie axial verjüngt auslaufen. Die Axialführung 3d dient der Linearführung des hinteren Gehäuseabschnitts 11 bei dem Verbinden des Reservoirmoduls 10 mit dem Dosier- und Betätigungsmodul 30. Wie in Figur 9 und am besten in Figur 11 zu erkennen ist ragen von einer Innenmantelfläche des hinteren Gehäuseabschnitts 11 der Anzahl nach entsprechend und der Form nach angepasst Eingriffselemente 11 d radial einwärts ab. In jede der Axialführungen 3d ragt ein Eingriffselement 11d ein und wird von der Axialführung 3d linear geführt, wenn der vordere Gehäuseabschnitt 1, 3 und der hintere Gehäuseabschnitt 11 zum Verbinden ineinander geschoben werden. Auf diese Weise wird sichergestellt, dass zwischen dem vorderen Gehäuseabschnitt 1, 3 und dem hinteren Gehäuseabschnitt 11 keine Relativdrehung stattfinden kann, wenn im Zuge des Verbindens der verdrehgesicherte Eingriff zwischen dem Dosier- und Betätigungselement 32 und dem Dosiseinstellglied 39 hergestellt wird.

Indem die Führungsrippen an ihren hinteren Enden axial verjüngt auslaufen und die Führungskanäle so zu Einführtrichtern verbreitert sind, wird eine Zentrierung zwischen dem vorderen Gehäuseabschnitt 1, 3 und dem hinteren Gehäuseabschnitt 11 zum Zwecke des Verbindens erleichtert. Die Führungsrippen laufen an ihren Enden auch radial zur Mantelfläche des Mechanikhalters 3 verjüngt aus, wodurch die Zentrierung der Gehäuseabschnitte 1, 3 und 11 in eine durch die Axialführung 3d vorgegebene Drehwinkelposition relativ zueinander noch mehr erleichtert wird.

Ebenso wie bei dem Ineinanderschieben der vordere Gehäuseabschnitt 1, 3 und der hintere Gehäuseabschnitt 11 an einer Relativdrehung zueinander gehindert werden, wird auch das Dosiseinstellglied 39 in Bezug auf seine Drehwinkelposition relativ zu dem vorderen Gehäuseabschnitt 1, 3 fixiert, wobei diese Fixierung lösbar ist, um die für die Dosierung erforderliche Drehbewegung des Dosiseinstellglieds 39 zuzulassen. Um daher zum einen die Dosierbewegung des Dosiseinstellglieds 39 zu ermöglichen, aber zum anderen eine unerwünschte Dosierbewegung durch das Herstellen der Verbindung zwischen dem vorderen Gehäuseabschnitt 1, 3 und dem hinteren Gehäuseabschnitt 11 zu verhindern, wird das Dosiseinstellglied 39 von dem Mechanikhalter 3 mittels einer lösbaren Rastverbindung in diskreten Drehwinkelpositionen fixiert.

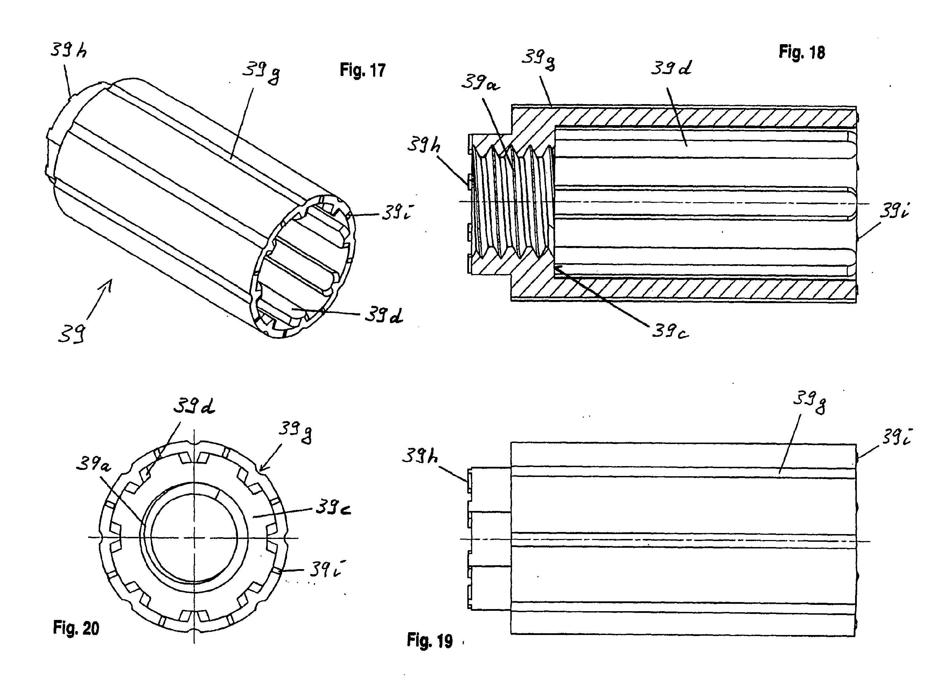

Die Figuren 17 bis 20 zeigen das Dosiseinstellglied 39 in Einzeldarstellungen. Für die Ausbildung der Rastverbindung sind an der Außenmantelfläche des Dosiseinstellglieds 39 über den Umfang in regelmäßiger Teilung verteilt mehrere Rastaufnahmen 39g ausgebildet. Jede der Rastaufnahmen 39g wird von einer geraden, axial sich erstreckenden Rinne mit einer im Querschnitt gerundet verlaufenden Kontur gebildet.

Der Mechanikhalter 3 ist mit zwei Rastnasen 3g (Figur 15 und 16) versehen. Die beiden Rastnasen 3g ragen in dem hinteren Hülsenabschnitt des Mechanikhalters 3 von einer Innenmantelfläche des Mechanikhalters 3 radial einwärts ab. Sie sind einander diametral gegenüberliegend angeordnet. Der jeweilige Mantelbereich des Mechanikhalters 3, an dem eine der Rastnasen 3g angeformt ist, bildet ein in radialer Richtung elastisch nachgiebiges Federelement 3f. Aufgrund der elastischen Nachgiebigkeit und der gerundeten Form der Rastnasen 3g in Verbindung mit dem gerundeten Profil der Rastaufnahmen 39g ist der Rasteingriff der Rastnasen 3g in die gegenüberliegenden Rastaufnahmen 39g lösbar. Die Lösbarkeit ist für die Dosisauswahl erforderlich. Andererseits ist der Rasteingriff jedoch so gestaltet, dass die Drehwinkelfixierung des Dosiseinstellglieds 39 so stabil ist, dass bei dem Verbinden des vorderen Gehäuseabschnitts 1, 3 mit dem hinteren Gehäuseabschnitt 11 keine unerwünschte Dosierbewegung des Dosiseinstellglieds 39 stattfinden kann, wenn die Drehkopplung des Dosier- und Betätigungselements 32 mit dem Dosier- und Betätigungselement 32 hergestellt wird. Die Rastverbindung zwischen dem Mechanikhalter 3 und dem Dosiseinstellglied 39 ergibt als vorteilhaften Nebeneffekt ein taktiles Signal bei der Dosierung. Um die günstige Elastizität des Federelements 3f zu erhalten, ist der hintere Hülsenabschnitt des Mechanikhalters 3 im betreffenden Mantelbereich ausgeschnitten, so dass das Federelement 3f als ein im Umfangsrichtung sich erstreckendes Ringsegment erhalten wird, das axial beidseits freiliegt.

Aus den Figuren 17, 18 und 20 ebenfalls erkennbar sind Axialführungen 39d für den verdrehsicheren Eingriff des Dosiseinstellglieds 39 mit dem Dosier- und Betätigungselement 32. Das Dosier- und Betätigungselement 32 ist mit wenigstens einem Eingriffselement versehen, um die axiale Linearführung, d.h. die Verdrehsicherung, zwischen dem Dosier- und Betätigungselement 32 und dem Dosiseinstellglied 39 zu erhalten. Die Axialführungen 39d sind wieder Führungskanäle, die von mehreren, axial sich gerade erstreckenden Führungsrippen gebildet werden. Jede der Führungsrippen läuft an ihrem hinteren, dem Dosier- und Betätigungselement 32 zugewandten Ende axial und radial verjüngt aus, um die Zentrierung zwischen dem Dosier- und Betätigungselement 32 und dem Dosiseinstellglied 39 bei der Herstellung des verdrehsicheren Eingriffs zu erleichtern. Für die axiale Linearführung von Dosiseinstellglied 39 und Dosier- und Betätigungselement 32 wird somit die gleiche Konstruktion wie für die axiale Linearführung der Gehäuseabschnitte 1, 3 und 11 verwendet.

Der Vollständigkeit wegen sei schließlich auch auf das Dosiergewinde 39a und den Ausschüttanschlag 39c des Dosiseinstellglieds 39 hingewiesen, die am besten in Figur 18 zu erkennen sind.

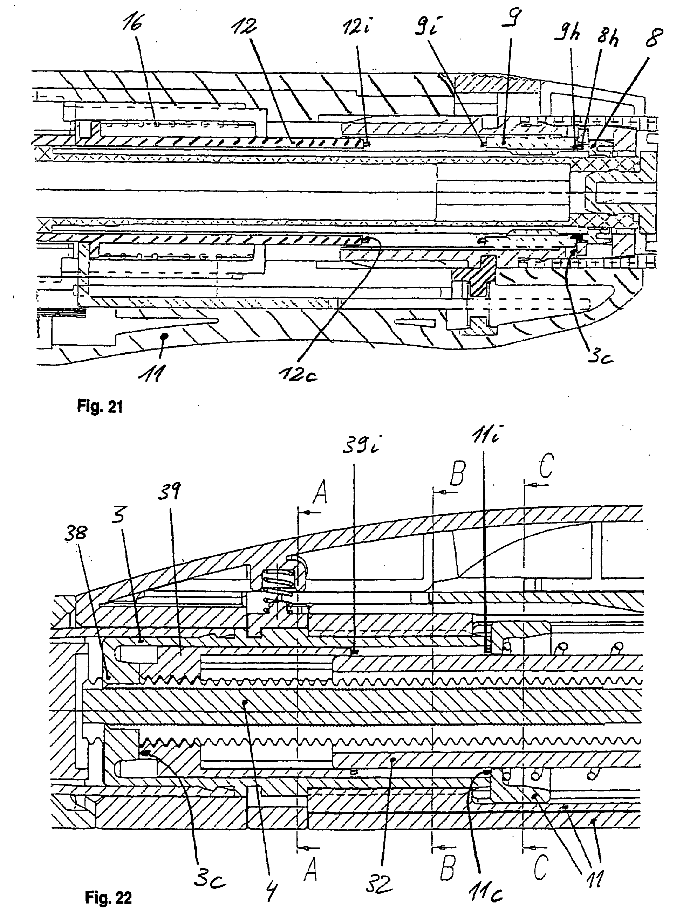

Schließlich sind für das Dosiseinstellglied 39 zwei Verdrehsicherungen vorgesehen, die in den beiden axialen Endpositionen des Dosiseinstellglieds 39 wirksam werden. Diesbezüglich sei ergänzend auf Figur 22 hingewiesen.

Um zu verhindern, dass die Kolbenstange 4 in Reaktion auf eine rotatorische Dosierbewegung des Dosiseinstellglieds 39 zurückbewegt werden könnte, sind an einem vorderen Ende des Dosiseinstellglieds 39 Verdrehanschläge 39h geformt. Die Verdrehanschläge 39h stehen in der vorderen Endposition, die das Dosiseinstellglied 39 unmittelbar nach einer Produktausschüttung bzw. vor einer Dosisauswahl einnimmt, mit Verdrehgegenanschlägen 3h in Eingriff, die an dem Mechanikhalter 3 angeformt sind (Fig. 16). Die Verdrehanschläge 39h ragen von einer vorderen Stirnseite des Dosiseinstellglieds 39 axial ab, und die Verdrehgegenanschläge 3h ragen von einer axial zugewandten Stirnfläche des Mechanikhalters 3, die den Aufschüttanschlag 3c bildet, den Verdrehanschlägen 39h axial entgegen. Der Eingriff zwischen den Verdrehanschlägen 39h und den Verdrehgegenanschlägen 3h ist derart, dass er eine rotatorische Dosierbewegung in eine Drehrichtung zulässt, die eine von dem Ausschüttanschlag 3c weg gerichtete translatorische Dosierbewegung des Dosiseinstellglieds 39 bewirkt, aber in der vorderen axialen Endposition eine rotatorische Dosierbewegung in die Gegendrehrichtung verhindert.

Ferner ist ein weiteres Paar von Verdrehanschlägen und Verdrehgegenanschlägen vorgesehen, die in grundsätzlich gleicher Weise wie die Anschläge 3h und 39h geformt sind und zusammenwirken. Bei diesem zweiten Verdrehanschlagpaar handelt es sich zum einen um Verdrehanschläge 39i, die von einer hinteren Stirnfläche des Dosiseinstellglieds 39 axial abragen, und zum anderen um Verdrehgegenanschläge 11 i, die von der zugewandten Anschlagstirnfläche des hinteren Translationsanschlags 11c axial auf das Dosiseinstellglied 39 zuragen, in der Figur 9 aufgrund ihrer geringen Abmessungen jedoch nicht erkennbar sind. Durch das hintere Verdrehanschlagpaar 11 i/39i wird in der hinteren Endposition verhindert, dass die Kolbenstange 4 in Reaktion auf eine gegen die Anschlagstirnfläche des hinteren Translationsanschlags 11 c gerichtete Dosierbewegung des Dosiseinstellglieds in Vorschubrichtung bewegt werden kann.
Die Höhe, d.h. die axiale Länge, sämtlicher Verdrehanschläge 3h, 39h, 11i und 39i ist auf die Gewindesteigung der im Eingriff befindlichen Dosiergewinde der Kolbenstange 4 und des Dosiseinstellglieds 39 abgestimmt. Die Verdrehanschläge sind axial so kurz, dass diejenige rotatorische Dosierbewegung nicht behindert wird, durch die das Dosiseinstellglied 39 von dem jeweiligen Translationsanschlag 3c oder 11c wegbewegt wird.

Bei der Montage der Komponenten des Reservoirmoduls 10 wird das Dosiseinstellglied 39 bis in eine vorgegebene Axialposition, wie sie aus Figur 9 ersichtlich ist, auf die Kolbenstange 4 aufgeschraubt. Anschließend wird die Kolbenstange 4 mit dem aufgeschraubten Dosiseinstellglied 39 von hinten in den Mechanikhalter 3 eingeführt, bis dessen Blockiereinrichtung 38 in Sperreingriff mit der Rückzugssperreinrichtung 6 der Kolbenstange 4 gelangt und ferner der verdrehgesicherte Eingriff zwischen den Verdrehanschlägen 39h des Dosiseinstellglieds 39 und den Verdrehgegenanschlägen 3h des Mechanikhalters 3 hergestellt ist. Bereits während der Einführung in den Mechanikhalter 3 wird das Dosiseinstellglied 39 in einer Drehwinkelrastposition durch den Rasteingriff zwischen den Rastnasen 3g und den Rastaufnahmen 39g von dem Mechanikhalter 3 axial linear geführt, bis das Dosiseinstellglied 39 an den Ausschüttanschlag 3c des Mechanikhalters 3 anstößt. In dieser vorderen Endposition des Dosiseinstellglieds 39 relativ zu dem Mechanikhalter 3 ist gleichzeitig auch bereits der verdrehsichere Eingriff zwischen den Verdrehanschlägen 3h und 39h hergestellt.

In diesem Zustand werden der Mechanikhalter 3 und ein bereits mit einem Reservoir ausgestattetes Reservoirteil 1 miteinander verbunden.

In einem nächsten Schritt wird der hintere Gehäuseabschnitt 11 des komplett montierten Dosier- und Betätigungsmoduls 30 auf den Mechanikhalter 3 geschoben, wobei sich der Mechanikhalter 3 und der hintere Gehäuseabschnitt 11 aufgrund der Axialführungen 3d und der Eingriffselemente 11 d des hinteren Gehäuseabschnitts 11 zueinander zentrieren können und nach der Zentrierung aufgrund des Führungseingriffs axial aneinander linear geführt werden. Im Zuge des Aufschiebens gelangt das Dosier- und Betätigungselement 32 in den verdrehgesicherten Eingriff mit dem Dosiseinstellglied 39, wobei auch hier durch eine den Axialführungen 3d und Eingriffselementen 11 d entsprechende Linearführung zuerst eine gewisse Zentrierung stattfinden kann.

Das Dosier- und Betätigungselement 32 ist in diskreten Drehwinkelrastpositionen mit dem hinteren Gehäuseabschnitt in einem Rasteingriff und wird in dem Rasteingriff, d.h. in der jeweiligen Drehwinkelrastposition, axial linear geführt. Die Drehwinkeldifferenz zwischen zwei aufeinanderfolgenden Drehwinkelrastpositionen entspricht einer Dosiseinheit. Die Linearführung zwischen dem Mechanikhalter 3 und dem hinteren Gehäuseabschnitt 11 einerseits und die diskreten Drehwinkelpositionen des Dosiseinstellglieds 39 relativ zu dem Mechanikhalter 3 (Rastnasen 3g und Rastaufnahmen 39g) und die Drehwinkelrastpositionen des Dosier- und Betätigungselements 32 relativ zu dem hinteren Gehäuseabschnitt 11 andererseits sind aufeinander so abgestimmt, dass ein lineares Übereinanderschieben der beiden Gehäuseabschnitte 1, 3 und 11 stets in solch einer Drehwinkelposition stattfindet, dass auch das Dosiseinstellglied 39 und das Dosier- und Betätigungselement 32 für ihren verdrehgesicherten Eingriff relativ zueinander ausgerichtet sind, so dass während des Verbindens des Reservoirmoduls 10 mit dem Dosier- und Betätigungsmodul 30 keine Relativdrehung zwischen den an der Dosierung beteiligten Komponenten stattfindet.

In Bezug auf die weiteren Details der Montage, insbesondere der Herstellung des Verriegelungseingriffs, und zur Funktionsweise des Injektionsgeräts nach dem zweiten Ausführungsbeispiel wird auf die Beschreibung zu dem ersten Ausführungsbeispiel verwiesen.

Auch bei dem Injekionsgerät nach dem ersten Ausführungsbeispiel können Verdrehsicherungen vorgesehen sein, die in den beiden axialen Endpositionen des Dosiseinstellglieds 9 des ersten Ausführungsbeispiels unerwünschte Reaktionsbewegungen der Kolbenstange 4 verhindern. Figur 21 zeigt die beiden Verdrehsicherungen, die in gleicher Weise wie die Verdrehsicherungen des zweiten Ausführungsbeispiels geformt sind. Allerdings werden die bei dem zweiten Ausführungsbeispiel an den Gehäuseabschnitten 1, 3 und 11 geformten Verdrehgegenanschläge bei dem ersten Ausführungsbeispiel zum einen von der Blockiereinrichtung 8 und zum anderen von dem Dosier- und Betätigungselement 12 gebildet. So sind an der Stirnfläche der Blockiereinrichtung 8, die dem Dosiseinstellglied 9 axial zugewandt ist, mehrere Verdrehanschläge 8h angeformt, die auf das Dosiseinstellglied 9 axial zu ragen. Da die Blockiereinrichtung 8 von dem vorderen Gehäuseabschnitt 1, 3 axial unbeweglich gelagert wird und mit der Kolbenstange 4 verdrehgesichert verbunden ist, wird auch durch das vordere Verdrehanschlagpaar 8h/9h eine Verdrehsicherung für die zwischen der Kolbenstange 4 und dem Dosiseinstellglied 9 stattfindende rotatorische Dosierbewegung erhalten. Das zweite Verdrehanschlagpaar ist zwischen dem Dosiseinstellglied 9 und dem hinteren Translationsanschlag 12c gebildet. Wie bei dem zweiten Ausführungsbeispiel ragen von der Stirnfläche des Translationsanschlags 12c, die dem Dosiseinstellglied 9 axial zugewandt ist, mehrere Verdrehanschläge 12i axial in Richtung auf das Dosiseinstellglied 9 vor. Das Dosiseinstellglied 9 ist wie bei dem zweiten Ausführungsbeispiel an seiner Rückseite mit Verdrehanschlägen 9i versehen, die in der hinteren axialen Endposition des Dosiseinstellglieds 9 in Eingriff mit den Verdrehanschlägen 12i gelangen. In der hinteren axialen Endposition des Dosiseinstellglieds 9 wird durch das hintere Verdrehanschlagpaar 9i/12i nur diejenige rotatorische Dosierbewegung zugelassen, die eine translatorische Dosierbewegung des Dosiseinstellglieds 9 in die Vorschubrichtung bewirkt.

### Bezugszeichen:

- 1: Reservoirteil, Ampullenhalter
- 2: Reservoir, Ampulle
- 3: Mechanikhalter
- 3a: Erstes Verriegelungselement
- 3b: Fixiereinrichtung
- 3c: Ausschüttanschlag, Translationsanschlag
- 3d: Axialführung
- 3e: Wulst
- 3f: Federelement
- 3g: Rastnase
- 3h: Verdrehanschlag
- 4: Kolbenstange
- 4a: Verbindungsabschnitt
- 5: Gewindeabschnitt
- 6: Rückzugsperreinrichtung, Zahnreihe
- 7: Vorschubbremseinrichtung, Zahnreihe
- 8: Blockiereinrichtung
- 8a: Sperrelement
- 8b: Bremselement
- 8h: Verdrehanschlag
- 9: Dosiseinstellglied
- 9h: Verdrehanschlag
- 9i: Verdrehanschlag
- 10: Reservoirmodul
- 11: hinterer Gehäuseabschnitt
- 11 d: Eingriffselement
- 11i: Verdrehanschlag
- 12: Dosier- und Betätigungselement
- 12i: Verdrehanschlag
- 13: Dosiermitnehmer
- 14: Verschluss
- 15a: Mitnehmer, Ringsteg
- 15b: Mitnehmer, Ringsteg
- 16: Rückstelleinrichtung
- 17: Zähl- und Anzeigeeinrichtung
- 18: -
- 19: -
- 20: Verriegelungsring
- 21: Zweites Verriegelungselement
- 22: Entriegelungsknopf
- 23: Sperrnocken
- 24: Rückstelleinrichtung
- 25: Verriegelungssperre
- 26: Mitnehmer, Steg
- 27: Axialausnehmung
- 28: Sperrzunge
- 29: Einrückausnehmung
- 30: Dosier- und Betätigungsmodul
- 31: Anschlagelement
- 32: Dosier- und Betätigungselement
- 33-37: -
- 38: Blockiereinrichtung
- 39: Dosiseinstellglied
- 39a: Dosiergewinde
- 39c: Ausschüttanschlag
- 39d: Axialführung
- 39g: Rastaufnahme, Axialführung
- 39h: Verdrehanschlag
- 39i: Verdrehanschlag

## Patentansprüche

1. Verabreichungsgerät für eine Verabreichung eines ausschüttbaren Produkts, das Gerät umfassend :
a) einen vorderen Gehäuseabschnitt (1,3), der ein Reservoir (2) für das Produkt umfasst,
b) einen hinteren Gehäuseabschnitt (11),
c) ein oder eine von einem Gehäuseabschnitt (1,3) gelagertes Abtriebsglied (4) oder Kolbenstange (4) zur Ausführung einer Ausschüttbewegung, durch die eine ausgewählte Produktdosis ausgeschüttet wird,
d) und ein Betätigungselement (12; 32), das relativ zu dem vorderen Gehäuseabschnitt (1,3) translatorisch bewegbar ist und bei der Herstellung einer Verbindung der Gehäuseabschnitte (1, 3, 11) mit dem Abtriebsglied (4) so gekoppelt wird, dass eine Translationsbewegung des Betätigungselements (12; 32) die Ausschüttbewegung des Abtriebsglieds (4) bewirkt,
e) ein Dosiseinstellglied (9; 39), das für eine Auswahl der Produktdosis relativ zu dem Abtriebsglied (4) eine Dosierbewegung ausführt;
f) wobei an einem (1, 3) der Gehäuseabschnitte (1, 3, 11) wenigstens eine Axialführung (3d) und an dem anderen (11) der Gehäuseabschnitte (1, 3, 11) wenigstens ein Eingriffselement (11d) gebildet sind,
**dadurch gekennzeichnet, dass** vorgesehen ist:
e1) dass das Dosiseinstellglied (9, 39) unmittelbar am Abtriebsglied (4) angreift;
d1) wobei das Betätigungselement ein Dosier- und Betätigungselement (12; 32) ist, das relativ zu dem vorderen Gehäuseabschnitt (1, 3) rotatorisch um eine Rotationsachse (L) und translatorisch bewegbar ist und bei der Herstellung einer Verbindung der Gehäuseabschnitte (1, 3, 11) mit dem Abtriebsglied (4) und dem Dosiseinstellglied (9; 39) so gekoppelt wird, dass eine Rotationsbewegung des Dosier- und Betätigungselementes (2; 32) die Dosierbewegung des Dosiseinstellglieds (9; 39) und eine Translationsbewegung des Dosier- und Betätigungselementes (12; 32) die Dosierbewegung des Dosiseinstellglieds (9; 39) und eine Translationsbewegung des Dosier- und Betätigungselementes (12; 32) die Ausschüttbewegung des Abtriebsglieds (4) bewirken,
f1) wobei die Axialführung (3d) und das Eingriffselement (11d) bei der Herstellung der Verbindung der Gehäuseabschnitte (1, 3, 11) ineinandergreifend eine Linearführung bilden, so dass die Gehäuseabschnitte (1,3, 11) bis in eine Verbindungsendstellung relativ zueinander um die Rotationsachse (L) nicht verdrehbar aufeinander geschoben werden.

2. Verabreichungsgerät nach Anspruch 1, **dadurch gekennzeichnet, dass** die wenigstens eine Axialführung (3d) an einer Mantelfläche des einen (1,3) der Gehäuseabschnitte (1, 3,11) gebildet ist.

3. Verabreichungsgerät nach dem vorhergehenden Anspruch, **dadurch gekennzeichnet, dass** an der Mantelfläche des einen (1, 3) der Gehäuseabschnitte (1, 3, 11) in Umfangsrichtung voneinander beabstandet mehrere Axialführungen (3d) gebildet sind.

4. Verabreichungsgerät nach einem der vorhergehenden Ansprüche, **dadurch gekennzeichnet, dass** das wenigstens eine Eingriffselement(11d) an einer Mantelfläche des anderen (11) der Gehäuseabschnitte (1, 3) gebildet ist.

5. Verabreichungsgerät nach einem der vorhergehenden Ansprüche, **dadurch gekennzeichnet, dass** die wenigstens eine Axialführung (3d) an einem Ende, das dem anderen (11) der Gehäuseabschnitte (1, 3, 11) zugewandt ist, in axialer Richtung und vorzugsweise auch in radialer Richtung verjüngt ausläuft, um eine Ausrichtung der Gehäuseabschnitte (1, 3, 11) zu erleichtern.

6. Verabreichungsgerät nach einem der vorhergehenden Ansprüche, dass das wenigstens eine Eingriffselement (11d) an einem Ende, das dem einen (1,3) der Gehäuseabschnitte (1, 3, 11) zugewandt ist, in axialer Richtung und vorzugsweise auch in radialer Richtung verjüngt ist, um eine Ausrichtung der Gehäuseabschnitte (1, 3, 11) zu erleichtern.

7. Verabreichungsgerät nach einem der vorhergehenden Ansprüche, **dadurch gekennzeichnet, dass** die Gehäuseabschnitte (1, 3, 11) in einer einzigen Drehwinkelposition oder in mehreren diskret vorgegebenen Drehwinkelpositionen aufeinander geschoben werden können.

8. Verabreichungsgerät nach dem vorhergehenden Anspruch, **dadurch gekennzeichnet, dass** die wenigstens eine Axialführung (3d) die Gehäuseabschnitte (1, 3, 11) in jeder der mehreren vorgegebenen Drehwinkelpositionen axial linear zueinander führt.

9. Verabreichungsgerät nach einem der zwei vorhergehenden Ansprüche, **dadurch gekennzeichnet, dass** die wenigstens eine Axialführung (3d) und/oder das wenigstens eine Eingriffselement (11d) verhindert, dass die Gehäuseabschnitte (1, 3, 11) in einer anderen Drehwinkelposition als der einzigen oder den mehreren vorgegebenen Drehwinkelpositionen aufeinander geschoben werden können.

10. Verabreichungsgerät nach einem der vorhergehenden Ansprüche, **dadurch gekennzeichnet, dass** das Dosiseinstellglied (9; 39) von einem (1, 3) der Gehäuseabschnitte (1,3, 11) axial linear geführt wird.

11. Verabreichungsgerät nach einem der vorhergehenden Ansprüche, **dadurch gekennzeichnet, dass** das Dosiseinstellglied (39) in vorgegebenen Drehwinkelpositionen mit einem (1, 3) der Gehäuseabschnitte (1, 3, 11) in einem lösbaren Eingriff ist und vorzugsweise in dem Eingriff axial linear geführt wird, wobei der Eingriff vorzugsweise ein Rasteingriff ist.

12. Verabreichungsgerät nach dem vorhergehenden Anspruch, **dadurch gekennzeichnet, dass** der lösbare Eingriff ein Rasteingriff ist, wobei wenigstens eine Rastnase (3g) und wenigstens eine Rastaufnahme (39g), von denen die eine an dem Dosiseinstellglied (39) und die andere an dem einen (1, 3) der Gehäuseabschnitte (1, 3; 11) gebildet ist, miteinander in dem Rasteingriff sind und gegen eine rückstellende Elastizitätskraft aus dem Rasteingriff bewegbar sind.

13. Verabreichungsgerät nach einem der vorhergehenden Ansprüche, **dadurch gekennzeichnet, dass** das Dosiseinstellglied (39) wenigstens einen Anschlag (39h ; 39i) aufweist, der in einer Dosierendstellung des Dosiseinstellglieds (39) mit einem der Gehäuseabschnitte (1, 3, 11) in einem Sperreingriff steht, der eine Bewegung des Dosiseinstellglieds (39) verhindert, die eine axiale Reaktionsbewegung des Abtriebsglieds (4) bewirken könnte.

14. Verabreichungsgerät nach einem der vorhergehenden Ansprüche, **dadurch gekennzeichnet, dass** der vordere Gehäuseabschnitt (1,3) ein erstes Verriegelungselement (3a) und der hintere Gehäuseabschnitt (11) ein zweites Verriegelungselement (21) umfasst und die Verriegelungselemente (3a, 21) in einem Verriegelungseingriff die Gehäuseabschnitte (1, 3, 11) axial aneinander fixieren.

15. Verabreichungsgerät nach einem der vorhergehenden Ansprüche, **dadurch gekennzeichnet, dass** die Dosierbewegung des Dosiseinstellglieds (9; 39) eine in Richtung der Rotationsachse (L) des Dosier- und Betätigungselementes (12; 32) weisende Translationsbewegung ist oder umfasst.

16. Verabreichungsgerät nach einem der vorhergehenden Ansprüche, **dadurch gekennzeichnet, dass** eine Rotationsbewegung, die das Abtriebsglied (4) und das Dosiseinstellglied (9; 39) relativ zueinander oder gemeinsam relativ zu wenigstens einem der Gehäuseabschnitte (1, 3, 11) ausführen, die Dosierbewegung des Dosiseinstellglieds (9; 39) bewirkt.

17. Verabreichungsgerät nach einem der vorhergehenden Ansprüche, **dadurch gekennzeichnet, dass** das Abtriebsglied (4) und das Dosiseinstellglied (9; 39) miteinander in einem Gewindeeingriff um eine in Richtung der Rotationsachse (L) des Dosier- und Betätigungselementes (12; 32) weisende Gewindelängsachse sind.

18. Verabreichungsgerät nach einem der vorhergehenden Ansprüche, **dadurch gekennzeichnet, dass** eine Kanüle von höchstens 30 Gauge, vorzugsweise eine 31 oder 32 Gauge Kanüle, oder eine Kanüle, die eine nicht in ISO-9626 spezifizierte Kombination von Aussendurchmesser und Innendurchmesser aufweist, mit einem Aussendurchmesser von höchstens 320 µm und einer möglichst dünnen Wandstärke eine Injektions- oder Infusionskanüle des Verabreichungsgeräts bildet.

19. Reservoirmodul zur Verbindung mit einem Verabreichungsgerät, das Reservoirmodul (10) umfassend:
a) einen vorderen Gehäuseabschnitt (1,3) des Verabreichungsgeräts, der ein Reservoir (2) für ein ausschüttbares Produkt umfasst,
b) einen Kolben, der in dem Reservoir (2) in eine Vorschubrichtung auf einen Auslass des Reservoirs (2) zu bewegbar aufgenommen ist, um Produkt auszuschütten,
c) und ein Abtriebsglied oder eine Kolbenstange (4), die so gehalten wird, dass eine Bewegung des Abtriebsgliedes oder der Kolbenstange (4) gegen die Vorschubrichtung verhindert wird,
**gekennzeichnet durch** folgende Merkmale:
d) ein Dosiseinstellglied (9; 39), das von dem vorderen Gehäuseabschnitt (1,3) bewegbar aufgenommen wird, um eine Dosierbewegung und eine Ausschüttbewegung auszuführen,
c1) wobei die Kolbenstange oder das Abtriebsglied (4), die mit dem Dosiseinstellglied (9; 39) verbunden ist, von dem vorderen Gehäuseabschnitt (1,3) so gehalten wird, dass eine Bewegung der Kolbenstange (4) oder des Abtriebsglieds gegen die Vorschubrichtung verhindert und in die Vorschubrichtung nicht **durch** die Dosierbewegung bewirkt wird,
e) wobei an einer Mantelfläche des vorderen Gehäuseabschnitts (1,3) für die Ausbildung einer Linearführung wenigstens eine Axialführung (3d) oder wenigstens ein Eingriffselement gebildet ist, um den vorderen Gehäuseabschnitt (1,3) und einen hinteren Gehäuseabschnitt des Verabreichungsgeräts bei der Herstellung einer Verbindung der Gehäuseabschnitte bis in eine Verbindungsendstellung verdrehgesichert aufeinander zu schieben.

20. Reservoirmodul nach dem vorhergehenden Anspruch, **dadurch gekennzeichnet, dass** das Dosiseinstellglied (39) in vorgegebenen Drehwinkelpositionen mit dem vorderen Gehäuseabschnitt (1,3) in einem lösbaren Eingriff ist und vorzugsweise in dem Eingriff axial lineargeführt wird, wobei der Eingriff vorzugsweise ein Rasteingriff ist.

21. Reservoirmodul nach dem vorhergehenden Anspruch, **dadurch gekennzeichnet, dass** der lösbare Eingriff ein Rasteingriff ist, wobei wenigstens eine Rastnase (3g) und wenigstens eine Rastaufnahme (39g), von denen die eine an dem vorderen Gehäuseabschnitt (1,3) und die andere an dem Dosiseinstellglied (39) ausgebildet ist, miteinander in dem Rasteingriff sind und gegen eine rückstellende Elastizitätskraft aus dem Rasteingriff bewegbar sind.

22. Reservoirmodul nach einem der vorhergehenden Ansprüche, **dadurch gekennzeichnet, dass** der vordere Gehäuseabschnitt (1,3) und das Dosiseinstellglied (39) je wenigstens einen Anschlag (3h, 39h) aufweisen und diese Anschläge (3h, 39h) in einer vorderen Dosierendstellung des Dosiseinstellglieds (39) in Eingriff sind, um eine Bewegung des Dosiseinstellglieds (39) zu verhindern, die eine Reaktionsbewegung der Kolbenstange (4) entgegen der Vorschubrichtung bewirken könnte.

23. Reservoirmodul nach einem der vorhergehenden Ansprüche, **dadurch gekennzeichnet, dass** der vordere Gehäuseabschnitt (1,3) ein hülsenförmiges Reservoirteil (1) mit dem Reservoir (2) und einen hülsenförmigen Mechanikhalter (3) umfasst, die separat gefertigt und vorzugsweise so miteinander verbunden sind, dass ein Benutzer die Verbindung zerstörungsfrei nicht lösen kann, wobei der Mechanikhalter (3) die Kolbenstange (4) hält.

24. Reservoirmodul nach dem vorhergehenden Anspruch, **dadurch gekennzeichnet, dass** der Mechanikhalter (3) einen Ausschüttanschlag (3c) für das Dosiseinstellglied (9 ; 39) bildet, um die Ausschüttbewegung zu begrenzen, und dass das Dosiseinstellglied (9 ; 39) durch seine Dosierbewegung entgegen der Vorschubrichtung von dem Ausschüttanschlag (3c) wegbewegt wird.

25. Reservoirmodul nach einem der zwei vorhergehenden Ansprüche, **dadurch gekennzeichnet, dass** der Mechanikhalter (3) eine Blockiereinrichtung(8 ; 38) umfasst und die Blockiereinrichtung (8 ; 38) und die Kolbenstange (4) in einem Sicherheitseingriff sind, der verhindert, dass die Kolbenstange (4) in eine Stellung rückführbar ist, die sie vor der Ausführung einer in die Vorschubrichtung gerichteten Bewegung eingenommen hat, und dass der Sicherheitseingriff nicht lösbar ist.

26. Reservoirmodul nach einem der vorhergehenden Ansprüche, **dadurch gekennzeichnet, dass** das Reservoirmodul (10) ein Verbrauchsmodul ist, dass nach einer Entleerung des Reservoirs (2) für einen Austausch im Ganzen vorgesehen ist.

## Claims

1. Administration device for an administration of a dispensable product, the device comprising:
a) a front housing section (1, 3) which comprises a reservoir (2) for the product,
b) a rear housing section (11),
c) a drive member (4) or piston rod (4), housed by a housing section (1, 3), to carry out a dispensing movement by which a selected product dose is dispensed,
d) and an actuator (12; 32) which can be moved translationally relative to the front housing section (1, 3) and, during the creation of a connection between the housing sections (1, 3, 11), is coupled with the drive member (4) such that a translational movement of the actuator (12; 32) effects the dispensing movement of the drive member (4),
e) a dose setting member (9; 39) which carries out a dosing movement relative to the drive member (4) to select the product dose;
f) wherein at least one axial guide (3d) is formed on one (1, 3) of the housing sections (1, 3, 11) and at least one engagement element (11 d) on the other (11) of the housing sections (1, 3, 11),
**characterized in that** it is provided that:
e1) the dose setting member (9, 39) engages directly on the drive member (4);
d1) wherein the actuator is a dosing and actuating element (12; 32) which can be moved rotationally about an axis of rotation (L) and translationally relative to the front housing section (1, 3) and, during the creation of a connection between the housing sections (1, 3, 11), is coupled with the drive member (4) and the dose setting member (9; 39) such that a rotational movement of the dosing and actuating element (2; 32) effects the dosing movement of the dose setting member (9; 39) and a translational movement of the dosing and actuating element (12; 32) effects the dosing movement of the dose setting member (9; 39) and a translational movement of the dosing and actuating element (12; 32) effects the dispensing movement of the drive member (4),
f1) wherein the axial guide (3d) and the engagement element (11d) interlock during the creation of the connection of the housing sections (1, 3, 11) to form a linear guide, with the result that the housing sections (1, 3, 11) are pushed non-rotatable on each other about the axis of rotation (L) into a connecting end position relative to each other.

2. Administration device according to claim 1, **characterized in that** the at least one axial guide (3d) is formed on a casing surface of the one (1, 3) housing section (1, 3, 11).

3. Administration device according to the previous claim, **characterized in that** several axial guides (3d) are formed at intervals in circumferential direction on the casing surface of the one (1, 3) housing section (1, 3, 11).

4. Administration device according to one of the previous claims, **characterized in that** the at least one engagement element (11 d) is formed on a casing surface of the other (11) housing section (1, 3).

5. Administration device according to one of the previous claims, **characterized in that** the at least one axial guide (3d) ends tapered at one end which faces the other (11) housing section (1, 3, 11) in axial direction and preferably also in radial direction, to facilitate an alignment of the housing sections (1, 3, 11).

6. Administration device according to one of the previous claims, **characterized in that** the at least one engagement element (11 d) is tapered at one end which faces the one (1, 3) housing section (1, 3, 11), in axial direction and preferably also in radial direction, to facilitate an alignment of the housing sections (1, 3, 11).

7. Administration device according to one of the previous claims, **characterized in that** the housing sections (1, 3, 11) can be pushed onto each other in a single rotational angle position or in several discretely predefined rotational angle positions.

8. Administration device according to the previous claim, **characterized in that** the at least one axial guide (3d) guides the housing sections (1, 3, 11) axially linear relative to one another in each of the several predefined rotational angle positions.

9. Administration device according to one of the two previous claims, **characterized in that** the at least one axial guide (3d) and/or the at least one engagement element (11 d) prevents the housing sections (1, 3, 11) from being able to be pushed onto one another in a rotational angle position other than the single or several predefined rotational angle positions.

10. Administration device according to one of the previous claims, **characterized in that** the dose setting member (9; 39) is guided axially linearly by one (1, 3) of the housing sections (1, 3, 11).

11. Administration device according to one of the previous claims, **characterized in that** in predefined rotational angle positions the dose setting member (39) is in a releasable engagement with one (1, 3) of the housing sections (1, 3, 11) and is preferably axially linearly guided in the engagement, wherein the engagement is preferably a catching engagement.

12. Administration device according to the previous claim, **characterized in that** the releasable engagement is a catching engagement, wherein at least one catch nose (3g) and at least one catch channel (39g), one of which is formed on the dose setting member (39) and the other on one (1, 3) of the housing sections (1, 3; 11), are in catching engagement with each other and can be moved out of the catching engagement against a restoring elastic force.

13. Administration device according to one of the previous claims, **characterized in that** the dose setting member (39) has at least one stop (39h; 39i) which in a dosing end position of the dose setting member (39) is in arresting engagement with one of the housing sections (1, 3, 11), which prevents a movement of the dose setting member (39) which could effect an axial reaction movement of the drive member (4).

14. Administration device according to one of the previous claims, **characterized in that** the front housing section (1, 3) comprises a first locking element (3a) and the rear housing section (11) a second locking element (21) and the locking elements (3a, 21) fix the housing sections (1, 3, 11) axially against one another in a locking engagement.

15. Administration device according to one of the previous claims, **characterized in that** the dosing movement of the dose setting member (9; 39) is or comprises a translational movement pointing in the direction of the axis of rotation (L) of the dosing and actuating element (12; 32).

16. Administration device according to one of the previous claims, **characterized in that** a rotational movement which the drive member (4) and the dose setting member (9; 39) carry out relative to each other or jointly relative to at least one of the housing sections (1, 3, 11) effects the dosing movement of the dose setting member (9; 39).

17. Administration device according to one of the previous claims, **characterized in that** the drive member (4) and the dose setting member (9; 39) are in threaded engagement with each other about a threaded longitudinal axis pointing in the direction of the axis of rotation (L) of the dosing and actuating element (12; 32).

18. Administration device according to one of the previous claims, **characterized in that** a cannula of at most 30 gauge, preferably a 31- or 32-gauge cannula, or a cannula which has a combination not specified in ISO-9626 of external and internal diameter, with an external diameter of at most 320 µm and a wall thickness that is as thin as possible forms an injection or infusion cannula of the administration device.

19. Reservoir module for connection to an administration device, the reservoir module (10) comprising:
a) a front housing section (1, 3) of the administration device which comprises a reservoir (2) for a dispensable product,
b) a piston which is housed movable in a feed direction towards an outlet of the reservoir (2) in the reservoir (2), in order to dispense product,
c) and a drive member or a piston rod (4), which is held such that a movement of the drive member or the piston rod (4) against the feed direction is prevented,
**characterized by** the following features:
d) a dose setting member (9; 39) which is housed movable by the front housing section (1, 3) in order to carry out a dosing movement and a dispensing movement,
e1) wherein the piston rod or the drive member (4), which is connected to the dose setting member (9; 39), is held by the front housing section (1, 3) such that a movement of the piston rod (4) or of the drive member against the feed direction is prevented and is not effected in the feed direction by the dosing movement,
e) wherein to form a linear guide at least one axial guide (3d) or at least one engagement element is formed on a casing surface of the front housing section (1, 3), in order to push the front housing section (1, 3) and a rear housing section of the administration device onto each other in rotation-resistant manner into a connection end position during the creation of a connection between the housing sections.

20. Reservoir module according to the previous claim, **characterized in that** in predefined rotational angle positions the dose setting member (39) is in a releasable engagement with the front housing section (1, 3) and is preferably axially linearly guided in the engagement, wherein the engagement is preferably a catching engagement.

21. Reservoir module according to the previous claim, **characterized in that** the releasable engagement is a catching engagement, wherein at least one catch nose (3g) and at least one catch channel (39g), one of which is formed on the front housing section (1, 3) and the other on the dose setting member (39), are in catching engagement with each other and can be moved out of the catching engagement against a restoring elastic force.

22. Reservoir module according to one of the previous claims, **characterized in that** the front housing section (1, 3) and the dose setting member (39) each have at least one stop (3h, 39h) and these stops (3h, 39h) are in engagement in a front dosing end position of the dose setting member (39), in order to prevent a movement of the dose setting member (39) which could effect a reaction movement of the piston rod (4) against the feed direction.

23. Reservoir module according to one of the previous claims, **characterized in that** the front housing section (1, 3) comprises a sleeve-shaped reservoir section (1) with the reservoir (2) and a sleeve-shaped mechanism holder (3) which are made separately and preferably connected with one another such that a user cannot release the connection without destroying it, wherein the mechanism holder (3) holds the piston rod (4).

24. Reservoir module according to the previous claim, **characterized in that** the mechanism holder (3) forms a dispensing stop (3c) for the dose setting member (9; 39) in order to delimit the dispensing movement, and **in that** the dose setting member (9; 39) is moved by its dosing movement away from the dispensing stop (3c) against the feed direction.

25. Reservoir module according to one of the two previous claims, **characterized in that** the mechanism holder (3) comprises a blocking device (8; 38) and the blocking device (8; 38) and the piston rod (4) are in a safety engagement which prevents the piston rod (4) from being returnable to a position which it occupied before carrying out a movement directed in feed direction, and **in that** the safety engagement is not releasable.

26. Reservoir module according to one of the previous claims, **characterized in that** the reservoir module (10) is a disposable module which is provided for complete replacement after the reservoir (2) has been emptied.

## Revendications

1. Appareil d'administration servant à administrer un produit déversable, l'appareil comprenant :
a) une section de boîtier avant (1,3) qui comprend un réservoir (2) pour le produit,
b) une section de boîtier arrière (11),
c) un élément de sortie (4) ou une tige du piston (4) logé(e) dans une section de boîtier (1,3) pour réaliser un mouvement de déversement par lequel la dose de produit sélectionnée est déversée,
d) et un élément d'actionnement (12 ; 32) qui peut être déplacé de manière translatoire par rapport à la section de boîtier avant (1,3) et lors de l'établissement d'une connexion des sections de boîtier (1, 3, 11), est couplé à l'élément de sortie (4) de sorte qu'un mouvement translatoire de l'élément d'actionnement (12 ; 32) provoque le mouvement de déversement de l'élément de sortie (4),
e) un élément de réglage du dosage (9 ; 39) qui réalise un mouvement de dosage par rapport à l'élément de sortie (4) pour une sélection de la dose de produit,
f) sur l'une (1, 3) des sections de boîtier (1, 3, 11) au moins un guidage axial (3d) et sur l'autre (11) section de boîtier (1,3, 11) au moins un élément d'engagement (11d) étant formés,
**caractérisé en ce qu'**il est prévu :
e1) que l'élément de réglage du dosage (9, 39) soit appliqué directement sur l'élément de sortie (4),
d1) l'élément d'actionnement étant un élément de dosage et d'actionnement (12 ; 32) qui peut être déplacé par rapport à la section de boîtier avant (1, 3) de manière à tourner autour d'un axe de rotation (L) et de manière translatoire et qui, lors de l'établissement d'une connexion des sections de boîtiers (1, 3, 11), est couplé à l'élément de sortie (4) et à l'élément de réglage du dosage (9 ; 39) de sorte qu'un mouvement de rotation de l'élément de dosage et d'actionnement (2 ; 32) provoque le mouvement de dosage de l'élément de réglage du dosage (9 ; 39) et un mouvement translatoire de l'élément de dosage et d'actionnement (12 ; 32) provoque le mouvement de dosage de l'élément de réglage du dosage (9 ; 39) et un mouvement translatoire de l'élément de dosage et d'actionnement (12 ; 32) provoque le mouvement de déversement de l'élément de sortie (4),
f1) le guidage axial (3d) et l'élément d'engagement (11d) lors de l'établissement de la connexion des sections de boîtier (1, 3, 11) formant un guidage linéaire en s'engageant l'un dans l'autre de sorte que les sections de boîtiers (1, 3, 11) soient repoussées les unes sur les autres jusqu'à une position finale de connexion les unes par rapport aux autres de manière à ne pas pouvoir être tordues autour de l'axe de rotation (L).

2. Appareil d'administration selon la revendication 1, **caractérisé en ce que** le au moins un guidage axial (3d) est formé sur une surface enveloppe d'une (1, 3) des sections du boîtier (1, 3, 11).

3. Appareil d'administration selon la revendication précédente, **caractérisé en ce que**, sur la surface enveloppe d'une (1, 3) des sections du boîtier (1, 3, 11), plusieurs guidages axiaux (3d) sont formés à distance les uns des autres dans le sens périmètrique.

4. Appareil d'administration selon l'une des revendications précédentes, **caractérisé en ce que** le au moins un élément d'engagement (11d) est formé sur une surface enveloppe de l'autre (11) des sections de boîtier (1, 3).

5. Appareil d'administration selon l'une des revendications précédentes, **caractérisé en ce que** le au moins un guidage axial (3d) sur une extrémité qui est orientée vers l'autre (11) des sections de boîtier (1, 3, 11) se termine de manière effilée dans le sens axial et de préférence également dans le sens radial pour faciliter un alignement des sections de boîtiers (1, 3, 11).

6. Appareil d'administration selon l'une des revendications précédentes, en ce que le au moins un élément d'engagement (11d) sur une extrémité qui est orientée vers l'une (1, 3) des sections de boîtier (1, 3, 11) est effilé dans le sens axial et de préférence également dans le sens radial pour faciliter un alignement des sections de boîtier (1, 3, 11).

7. Appareil d'administration selon l'une des revendications précédentes, **caractérisé en ce que** les sections de boîtier (1, 3, 11) peuvent être repoussées les unes sur les autres dans une position unique d'angle de rotation ou dans plusieurs positions d'angle de rotation prescrites de façon discrète.

8. Appareil d'administration selon la revendication précédente, **caractérisé en ce que** le au moins un guidage axial (3d) guide, de manière axiale et linéaire, les sections de boîtier (1, 3, 11) les unes par rapport aux autres à chacune des plusieurs positions d'angle de rotation prescrites.

9. Appareil d'administration selon l'une des deux revendications précédentes, **caractérisé en ce que** le au moins un guidage axial (3d) et/ou le au moins un élément d'engagement (11d) empêchent que les sections de boîtier (1, 3, 11) puissent être repoussées les unes sur les autres dans une autre position d'angle de rotation que la position d'angle de rotation unique ou que les autres positions d'angle de rotation prescrites.

10. Appareil d'administration selon l'une des revendications précédentes, **caractérisé en ce que** l'élément de réglage du dosage (9 ; 39) est guidé de manière axiale et linéaire par l'une (1, 3) des sections de boîtier (1, 3, 11).

11. Appareil d'administration selon l'une des revendications précédentes, **caractérisé en ce que** l'élément de réglage du dosage (39) est engagé de manière détachable dans des positions d'angle de rotation prescrites avec l'une (1, 3) des sections de boîtier (1, 3, 11) et de préférence est guidé de manière axiale et linéaire dans l'engagement, l'engagement étant de préférence un engagement à encliquetage.

12. Appareil d'administration selon la revendication précédente, **caractérisé en ce que** l'engagement détachable est un engagement à encliquetage, au moins un nez d'encliquetage (3g) et au moins un logement d'encliquetage (39g) parmi lesquels l'un est formé sur l'élément de réglage du dosage (39) et l'autre sur l'une (1, 3) des sections de boîtier (1, 3 ; 11), étant ensemble dans l'engagement à encliquetage et pouvant être déplacés de l'engagement à encliquetage contre une force d'élasticité pouvant être réinitialisée.

13. Appareil d'administration selon l'une des revendications précédentes, **caractérisé en ce que** l'élément de réglage du dosage (39) présente au moins une butée (39h ; 39i) qui se trouve à une position finale de dosage de l'élément de réglage du dosage (39) avec l'une des sections de boîtier (1, 3, 11) dans un engagement de blocage, qui empêche un mouvement de l'élément de réglage du dosage (39) qui pourrait provoquer un mouvement de réaction axial de l'élément de sortie (4).

14. Appareil d'administration selon l'une des revendications précédentes, **caractérisé en ce que** la section de boîtier avant (1,3) comprend un premier élément de verrouillage (3a) et la section de boîtier arrière (11) un deuxième élément de verrouillage (21) et les éléments de verrouillage (3a, 21) fixent de manière axiale les sections de boîtier (1, 3, 11) les unes aux autres dans un engagement de verrouillage.

15. Appareil d'administration selon l'une des revendications précédentes, **caractérisé en ce que** le mouvement de dosage de l'élément de réglage du dosage (9 ; 39) est ou comprend un mouvement translatoire indiquant l'axe de rotation (L) de l'élément de dosage et d'actionnement (12 ; 32).

16. Appareil d'administration selon l'une des revendications précédentes, **caractérisé en ce qu'**un mouvement de rotation que réalise l'élément de sortie (4) et l'élément de réglage du dosage (9 ; 39) l'un par rapport à l'autre ou ensemble par rapport à au moins l'une des sections de boîtier (1, 3, 11), provoque le mouvement de dosage de l'élément de réglage du dosage (9 ; 39).

17. Appareil d'administration selon l'une des revendications précédentes, **caractérisé en ce que** l'élément de sortie (4) et l'élément de réglage du dosage (9 ; 39) sont l'un avec l'autre dans un engagement taraudé autour d'un axe longitudinal de taraudage indiquant l'axe de rotation (L) de l'élément de dosage et d'actionnement (12 ; 32).

18. Appareil d'administration selon l'une des revendications précédentes, **caractérisé en ce qu'**une canule de calibre 30 maximum, de préférence une canule de calibre 31 ou 32, ou une canule qui présente une combinaison de diamètre externe et interne non spécifiée dans la norme ISO-9626, avec un diamètre externe de 320 µm maximum et une épaisseur de paroi la plus mince possible forme une canule d'injection ou de perfusion de l'appareil d'administration.

19. Module de réservoir destiné à être raccordé à un appareil d'administration, le module de réservoir (10) comprenant :
a) une section de boîtier avant (1,3) de l'appareil d'administration qui comprend un réservoir (2) pour un produit déversable,
b) une tige qui est logée de manière à pouvoir être déplacée dans le réservoir (2) dans une direction d'avance vers une évacuation du réservoir (2) pour déverser le produit,
c) et un élément de sortie ou une tige de piston (4) qui est maintenue de sorte qu'un mouvement de l'élément de sortie ou de la tige de piston (4) contre la direction de l'avance soit empêché,
**caractérisé par** les caractéristiques suivantes :
d) un élément de réglage du dosage (9 ; 39) qui est logé de manière à pouvoir être déplacé dans la section de boîtier avant (1,3) pour réaliser un mouvement de dosage et un mouvement de déversement,
c1) la tige de piston ou l'élément de sortie (4) qui est raccordé à l'élément de réglage du dosage (9 ; 39), étant maintenu par la section de boîtier avant (1,3) de sorte qu'un mouvement de la tige de piston (4) ou de l'élément de sortie contre la direction de l'avance soit empêché et ne soit pas provoqué par le mouvement de dosage dans la direction de l'avance,
e) sur une surface enveloppe de la section de boîtier avant (1,3) pour la formation d'un guidage linéaire, au moins un guidage axial (3d) ou au moins un élément d'engagement étant formé pour repousser l'une sur l'autre la section de boîtier avant (1,3) et la section de boîtier arrière de l'appareil d'administration lors de l'établissement d'une connexion des sections de boîtier jusqu'à une position finale de connexion de manière protégée contre la torsion.

20. Module de réservoir selon la revendication précédente, **caractérisé en ce que** l'élément de réglage du dosage (39) aux positions d'angle de rotation prescrites est dans un engagement détachable avec la section de boîtier avant (1,3) et de préférence est guidé de manière axiale et linéaire dans l'engagement, l'engagement étant de préférence un engagement à encliquetage.

21. Module de réservoir selon la revendication précédente, **caractérisé en ce que** l'engagement détachable est un engagement à encliquetage, au moins un nez d'encliquetage (3g) et au moins un logement d'encliquetage (39g) parmi lesquels l'un est formé sur la section de boîtier avant (1,3) et l'autre sur l'élément de réglage du dosage (39), étant ensemble dans l'engagement à encliquetage et pouvant être déplacés de l'engagement à encliquetage contre une force d'élasticité pouvant être réinitialisée.

22. Module de réservoir selon l'une des revendications précédentes, **caractérisé en ce que** la section de boîtier avant (1,3) et l'élément de réglage du dosage (39) présentent chacun au moins une butée (3h, 39h) et ces butées (3h, 39h) sont engagées à une position finale de dosage avant de l'élément de réglage du dosage (39) pour empêcher un mouvement de l'élément de réglage du dosage (39) qui pourrait provoquer un mouvement de réaction de la tige de piston (4) contraire à la direction de l'avance.

23. Module de réservoir selon l'une des revendications précédentes, **caractérisé en ce que** la section de boîtier avant (1,3) comprend une partie de réservoir (1) en forme de douille avec le réservoir (2) et un support mécanique (3) en forme de douille qui sont fabriqués séparément et de préférence reliés ensemble de sorte qu'un utilisateur ne puisse détacher le raccordement de manière non destructive, le support mécanique (3) maintenant la tige de piston (4).

24. Module de réservoir selon la revendication précédente, **caractérisé en ce que** le support mécanique (3) forme une butée de déversement (3c) pour l'élément de réglage du dosage (9 ; 39) afin de limiter le mouvement de déversement et **en ce que** l'élément de réglage du dosage (9 ; 39) est déplacé par son mouvement de dosage contraire à la direction de l'avance de la butée de déversement (3c).

25. Module de réservoir selon l'une des deux revendications précédentes, **caractérisé en ce que** le support mécanique (3) comprend un dispositif de blocage (8 ; 38) et le dispositif de blocage (8 ; 38) et la tige de piston (4) sont dans un engagement de sécurité qui empêche que la tige de piston (4) puisse retourner à une position qu'elle a occupée avant la réalisation d'un mouvement dirigé dans la direction de l'avance et **en ce que** l'engagement de sécurité n'est pas relâchable.

26. Module de réservoir selon l'une des revendications précédentes, **caractérisé en ce que** le module de réservoir (10) est un module de consommation qui est prévu dans son ensemble pour un échange après un vidage du réservoir (2).
